# EUROPÄISCHE PATENTSCHRIFT

(11) **EP 1 943 211 B1**
(45) Veröffentlichungstag und Bekanntmachung des Hinweises auf die Patenterteilung: **17.08.2011**
(21) Anmeldenummer: 06840923.4
(22) Anmeldetag: 30.10.2006
(51) Int. Cl.: C07C 213/08, C07C 217/62

(54) **VERFAHREN ZUR HERSTELLUNG SUBSTITUIERTER DIMETHYL-(3-ARYL-BUTYL)-AMIN-VERBINDUNGEN MITTELS HOMOGENER KATALYSE**
PROCESS FOR PREPARING SUBSTITUTED DIMETHYL(3-ARYLBUTYL)AMINE COMPOUNDS BY MEANS OF HOMOGENEOUS CATALYSIS
PROCEDE DE FABRICATION DE COMPOSES DIMETHYL-(3-ARYLBUTYL)AMINE SUBSTITUES A L AIDE D UNE CATALYSE HOMOGENE

(30) Priorität: 02.11.2005 DE 102005052588
(43) Veröffentlichungstag der Anmeldung: 16.07.2008
(73) Patentinhaber: Grünenthal GmbH, 52078 Aachen (DE)
(72) Erfinder: HELL, Wolfgang, 52066 Aachen (DE); KEGEL, Markus, 52078 Aachen (DE); BUSCHMANN, Helmut, Barcelona (ES); SPINDLER, Felix, CH-4656 Starrkirch-Wil (CH); HELLER, Detlef, 18057 Rostock (DE); DREXLER, Hans-Joachim, 18198 Gross-Schwass (DE)
(74) Vertreter: Brosch, Oliver
(86) Internationale Anmeldenummer: PCT/EP2006/010407
(87) Internationale Veröffentlichungsnummer: WO 2007/051576

(56) Entgegenhaltungen:
- EP-A1- 0 612 758
- EP-A1- 0 646 590
- EP-A1- 0 693 475
- EP-A1- 0 729 969
- EP-A2- 0 728 768
- WO-A-2004/108658
- WO-A-2005/000788
- WO-A2-01/49651
- WILLIAM D. LIBELL ET AL: "alpha-Amino Acids as Chiral Educts for Asymmetric Products. Alkylation of N-Phenylfluorenyl alpha-Amino Ketones. Synthesis of Optically Pure alpha-Alkyl Carboxylic Acids" J. AM. CHEM. SOC., Bd. 110, Nr. 22, 1988, Seiten 7447-7455, XP002419870

## Beschreibung

Die vorliegende Erfindung betrifft ein Verfahren zur Herstellung substituierter Dimethyl-(3-aryl-butyl)-amin-Verbindungen mittels homogener, katalytischer Hydrierung von Dimethyl-(3-aryl-but-3-enyl)-aminen.

Dimethyl-(3-aryl-butyl)-amin-Verbindungen haben sich als pharmazeutische Wirkstoffe mit exzellenter analgetischer Wirksamkeit und sehr guter Verträglichkeit erwiesen, siehe EP-A1-0 693 475. In der WO 2005/000788 A1 wird ein Verfahren zu deren Herstellung beschrieben, bei dem in einer ersten Stufe zunächst substituierte Dimethyl-(3-aryl-but-3-enyl)-amin-Verbindungen durch Eliminierung der tertiären Hydroxylgruppe in substituierten 4-Dimethylami-no-2-aryl-butan-2-ol-Verbindungen hergestellt werden. Diese Dimethyl-(3-aryl-but-3-enyl)-amin-Verbindungen werden dann in einer zweiten Stufe in Gegenwart von Metallkatalysatoren hydriert. Die heterogene Hydrierung verläuft bei ausreichenden Aktivitäten in guten Ausbeuten. Die Stereoselektivität ist erwartungsgemäss nicht sehr ausgeprägt. Gemäss den Beispielen in der WO 2005/000788 A1 können bei Anwesenheit von zwei benachbarten asymmetrischen C-Atomen Diastereomerenverhältnisse von 2:1 bis maximal 3:1 für trans-Diastereomer : cis- Diastereomer erhalten werden, also stets zugunsten des trans-Diastereomeren. Das Verhältnis stellt sich von selbst ein, ist im wesentlichen vom Substrat abhängig und kann nur im geringen Umfang durch die Wahl von Reaktionsbedingungen beeinflusst werden.

Weitere Verfahren der heterogenen katalytischen Hydrierung sind in WO 01/49651 A2 und WO 2004/108658 A beschrieben. Aus dem Stand der Technik sind ferner Verfahren der homogen katalytischen Hydrierung bekannt (W. D. Libell et al., J. Am. Chem. Soc. 1988, 110 (22): 7447-7455; EP 0 646 590 A1; EP0 729 969 A1; EP 0 612 758 A1; EP 0 728 768 A2).

Aufgabe der vorliegenden Erfindung ist es, ein verbessertes und flexibleres Verfahren zur Herstellung substituierter Dimethyl-(3-aryl-butyl)-amin-Verbindungen zur Verfügung zu stellen. Es wurde nun gefunden, dass man Dimethyl-(3-aryl-but-3-enyl)-amin-Verbindungen in Gegenwart von löslichen Hydrierkatalysatoren auch in homogener Phase hydrieren und hierbei hohe Umsätze und Ausbeuten erzielen kann. Es wurde ferner gefunden, dass man auch die Stereoselektivität mit der Wahl von chiralen Liganden gezielt beeinflussen und sehr hohe optische Ausbeuten erzielen kann. Es wurde auch gefunden, dass man mit der Wahl von chiralen Liganden die gewünschte Konfiguration erhalten kann, wenn man vom gleichen substituierten Dimethyl-(3-aryl-but-3-enyl)-amin ausgeht.

Gegenstand der vorliegenden Erfindung ist ein Verfahren zur Herstellung einer substituierten Dimethyl-(3-aryl-butyl)-amin-Verbindung der allgemeinen Formel III, 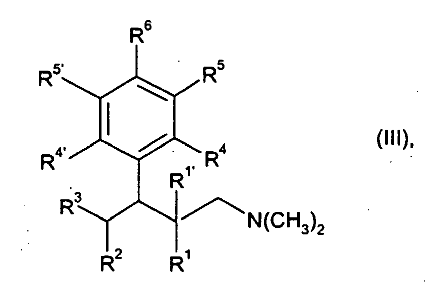 worin
R¹, R^{1'}, R², R³ unabhängig voneinander jeweils -H oder -C₁₋₅-Alkyl bedeuten,
R⁴, R^{4'}, R⁵, R^{5'}, R⁶, gleich oder verschieden, jeweils für,-H, -OH, -C₁₋₄-Alkyl, -O-C₁₋₄-Alkyl, teil-oder perfluoriertes -C₁₋₄-Alkyl, teil- oder perfluoriertes -O-C₁₋₄-Alkyl, -O-(CH₂)ₙ-Phenyl mit n gleich 1, 2 oder 3, F, Cl oder OR⁸ stehen, oder zwei benachbarte Reste R⁴ und R⁵, R⁵ und R⁶, R⁶ und R^{5'} oder R^{5'} und R^{4'} für eine Gruppe -OCH=CHO-, -CH=C(R⁹)-O-, -CH=C(R⁹)-S-oder -CH=CH-C(OR¹⁰)=CH- als Teil eines Ringes stehen, mit der Massgabe, dass die jeweils anderen Reste R⁶, R⁵ und R^{4'}, R⁴, R^{5"} und R^{6'}, R⁴, R⁵ und R^{4'} bzw. R⁴, R⁵ und R⁶ die vorstehend genannte Bedeutung haben,
R⁸ -CO-C₁₋₅-Alkyl, -PO(O-C₁₋₄-Alkyl)₂, -CO-C₆H₄-R¹¹, -CO(O-C₁₋₅-Alkyl), -CO-CHR¹²-NHR¹³, -CO-NH-C₆H₃-(R¹⁴)₂ oder eine unsubstituierte oder substituierte Pyridyl-, Thienyl-, Thiazoyl-oder Phenylgruppe bedeutet,
R⁹ -H oder -C₁₋₄-Alkyl bedeutet,
R¹⁰-H oder -C₁₋₃-Alkyl bedeutet,
R¹¹ -OC(O)-C₁₋₃-Alkyl in ortho-Stellung oder -CH₂-N-(R¹⁵)₂ in meta- oder para-Stellung, wobei R¹⁵ jeweils -C₁₋₄-Alkyl oder beide Reste R¹⁵ zusammen mit dem verbrückenden Stickstoffatom einen 4-Morpholino-Rest bilden,
R¹² und R¹³, gleich oder verschieden, jeweils für -H, -C₁₋₆-Alkyl oder -C₃₋₈-Cycloalkyl stehen, oder R¹² und R¹³ zusammen -(CH₂)₃₋₈ als Teil eines Rings bedeuten,
R¹⁴ -H, -OH, -C₁₋₇-Alkyl, teil- oder perfluoriertes -C₁₋₇-Alkyl, -OC₁₋₇-Alkyl, -Phenyl, -O-Aryl, -F oder -Cl mit der Massgabe, dass die Reste R¹⁴ gleich oder verschieden sind,
jeweils in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, gegebenenfalls in Form eines Salzes, eines Solvates oder in Form eines Salzes und Solvates,
das dadurch gekennzeichnet ist, dass man eine substituierte Dimethyl-(3-aryl-but-3-enyl)-amin-Verbindung der allgemeinen Formel II, 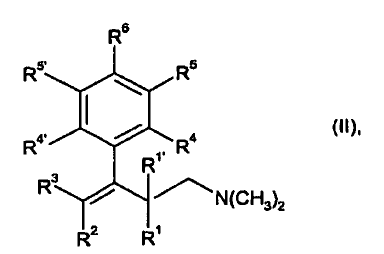 worin die Reste R¹, R^{1'}, R², R³, R⁴, R^{4'}, R⁵, R^{5'} und R⁶ jeweils die vorstehend genannte Bedeutung haben, jeweils in Form von Racematen, reinen Enantiomeren, Gemischen von Enantiomeren in einem beliebigen Mischungsverhältnis, Z- oder E-Isomeren oder Gemischen von Z- oder E-Isomeren in einem beliebigen Mischungsverhältnis, Salzen oder Solvaten, in Gegenwart von Wasserstoff und einem löslichen Rhodiumsalz oder Rhodiumkomplex als homogenem Katalysator zu einer Verbindung der allgemeinen Formel III umsetzt, wobei die als Katalysator verwendeten Rhodiumsalze oder Rhodiumkomplexe chirale Liganden mit zwei Sekundärphosphingruppen (Diphosphinligand) aufweisen.

Das erfindungsgemässe Verfahren ist besonders für asymmetrische Hydrierungen geeignet

Bevorzugt werden in dem erfindungsgemässen Verfahren substituierte Dimethyl-(3-aryl-but-3-enyl)-amin-Verbindungen der allgemeinen Formel IIA 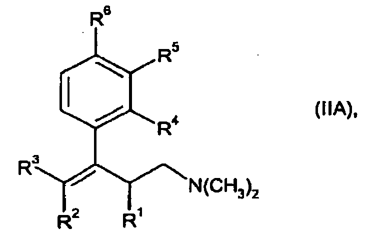 worin
R¹ -C₁₋₅-Alkyl ist,
R² -H oder -C₁₋₅-Alkyl bedeutet,
R³ -H oder -C₁₋₅-Alkyl bedeutet,
R⁴ -H, -OH, -C₁₋₄-Alkyl, -O-C₁₋₄-Alkyl, -O-Benzyl, -CF₃, -O-CF₃, -Cl, -F oder -OR⁸ ist,
R⁵ -H, -OH, -C_{'-4}-Alkyl, -O-C₁₋₄-Alkyl, -O-Benzyl, -CHF₂, -CF₃, -O-CF₃, -Cl, -F oder -OR⁸ ist,
R⁶ -H, -OH, -C₁₋₄₋Alkyl, -O-C₁₋₄-Alkyl, -O-Benzyl, -CF₃, -O-CF_{3,} -Cl, -F oder -OR⁸ bedeutet,
mit der Massgabe, dass zwei der Reste R⁴, R⁵ oder R⁶ -H sind, oder R⁴ und R⁵ zusammen eine Gruppe -CH=C(R⁹)-O- oder -CH=C(R⁹)-S- als Teil eines Ringes bedeuten, wobei R⁶ -H ist, oder R⁵ und R⁶ zusammen eine Gruppe -CH=CH-C(OR¹⁰)=CH- als Teil eines Ringes bedeuten, wobei R⁴ -H ist,
R⁸ -CO-C₁₋₅-Alkyl, -PO(O-C₁₋₄-Alkyl)₂, -CO-C₆H₄-R¹¹, -CO(O-C₁₋₅-Alkyl), -CO-CHR¹²-NHR¹³, -CO-NH-C₆H₃-(R¹⁴)₂ oder eine unsubstituierte oder substituierte Pyridyl-, Thienyl-, Thiazoyl-oder Phenylgruppe bedeutet,
R⁹ -H oder -C₁₋₄-Alkyl bedeutet, R¹⁰ -H oder C₁₋₃-Alkyl bedeutet,
R¹¹ -OC(O)- C₁₋₃-Alkyl in ortho-Stellung oder -CH₂-N-(R¹⁵)₂ in meta- oder para Stellung, wobei R¹⁵ -C₁₋₄-Alkyl ist oder beide Reste R¹⁵ zusammen mit dem verbrückenden Stickstoffatom einen 4-Morpholino-Rest bilden, bedeutet,
R¹² und R¹³, gleich oder verschieden, jeweils für -H, -C₁₋₆-Alkyl oder -C₃₋₈-Cycloalkyl stehen, oder R¹² und R¹³ zusammen -(CH₂)₃₋₈ als Teil eines Rings bedeuten,
R¹⁴ -H, -OH, -C₁₋₇-Alkyl, -O-C₁₋₇-Alkyl, -Phenyl, -O-Aryl, -CF₃, -Cl oder -F bedeutet, mit der Massgabe, dass die beiden Reste R¹⁴ gleich oder verschieden sind.

Besonders bevorzugt werden in dem erfindungsgemässen Verfahren substituierte Dimethyl-(3-aryl-but-3-enyl)-amin-Verbindungen der allgemeinen Formel IIA verwendet, in denen
R¹ -C₁₋₃-Alkyl,
R² -H oder C₁₋₃-Alkyl,
R³ -H oder C₁₋₃-Alkyl,
R⁴ -H -OH, -Cl, -F oder -OR⁸
R⁵ -H, -OH, -C₁₋₄-Alkyl, -o-C₁₋₄-Alkyl, -O-Benzyl, -CHF₂, -CF₃, -Cl, -F oder -OR⁸,
R⁶ -H, -OH, -O- C₁₋₄-Alkyl, -O-Benzyl, -CF₃, -Cl, -F oder -OR⁸ bedeutet,
mit der Massgabe, dass zwei der Reste R⁴, R⁵ oder R⁶ -H sind, oder R⁴ und R⁵ zusammen eine Gruppe -CH=C(R⁹)-O- oder -CH=C(R⁹)-S- als Teil eines Ringes bedeuten, mit der Massgabe, dass R⁶ -H ist, oder R⁵ und R⁶ zusammen eine Gruppe -CH=CH-C(OR¹⁰)=CH-als Teil eines Ringes bedeuten, mit der Massgabe, dass R⁴ -H ist, und R⁸ bis R¹⁰ die vorstehend genannte Bedeutung haben.

Ganz besonders bevorzugt kommen in dem erfindungsgemässen Verfahren substituierte Dimethyl-(3-aryl-but-3-enyl)-amin-Verbindungen der allgemeinen Formel IIA zum Einsatz, in denen
R¹ -CH₃ oder -C₃H₇ ist,
R² -H, -CH₃ oder -CH₂CH₃ ist,
R³ -H, -CH₃ oder -CH₂CH₃ ist,
R⁴ -H oder -OH ist,
R⁵ -H, -OH, -OCH₃, -CHF₂ oder -OR⁸ ist,
R⁶ -H, -OH oder -CF₃ ist,
mit der Massgabe, dass zwei Reste R⁴, R⁵ oder R⁶ -H sind, oder R⁴ und R⁵ zusammen eine Gruppe -CH=C(CH₃)-S- als Teil eines Ringes darstellen, wobei R⁶ -H ist, oder R⁵ und R⁶ zusammen -CH=CH-C(OH)=CH- als Teil eines Ringes bedeuten, wobei R⁴ -H ist, R⁸ -CO-C₆H₄-R" und R" -OC(O)-C₁₋₃-Alkyl in ortho-Stellung bedeutet.

Ganz besonders bevorzugt kommen in dem erfindungsgemässen Verfahren substituierte Dimethyl-(3-aryl-but-3-enyl)-amin-Verbindungen der allgemeinen Formel IIA zum Einsatz, in denen R¹ und R³ jeweils -CH₃ und R⁵ -OCH₃ und die übrigen Reste ein Wasserstoffatom darstellen, entsprechend der nachfolgenden Formel IIB, oder das 2R-Enatiomere der Formel IIC

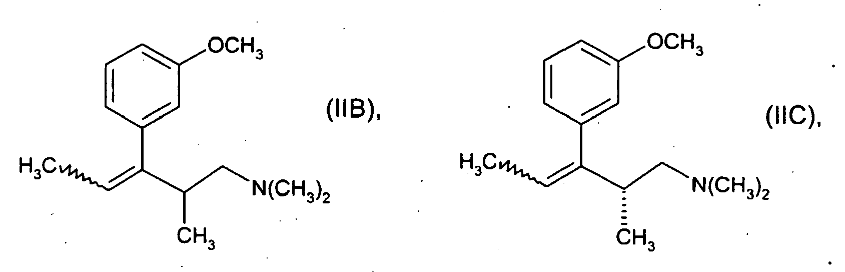

Am meisten bevorzugt wird die Verbindung der Formel IIC, also 3-[(3-Methoxy-phenyl)-2-methyl-pent-3-enyl]-dimethylamin, in Form eines seiner Cis- oder Transisomeren, nämlich (E)-(2R)-3-[(3-Methoxy-phenyl)-2-methyl-pent-3-enyl]-dimethylamin gemäss der Formel IIC.2 und insbesondere bevorzugt als (Z)-(2R)-3-[(3-Methoxy-phenyl)-2-methyl-pent-3-enyl]-dimethylamin gemäss der Formel IIC.1, oder Mischungen mit überwiegendem Anteil am Z-Isomer, eingesetzt:

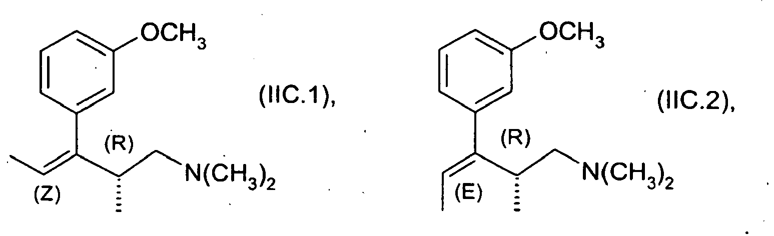

Ausgehend von Mischungen, bevorzugt jedoch von einem der isolierten Enantiomere, ist mit dem erfindungsgemässen Verfahren, unter Wahl entsprechend geeigneter chiraler Liganden für den homogenen Katalysator, die weitgehend stereoselektive Herstellung eines der zwei Diastereomere der Formeln III.C1 oder III.C2 möglich:

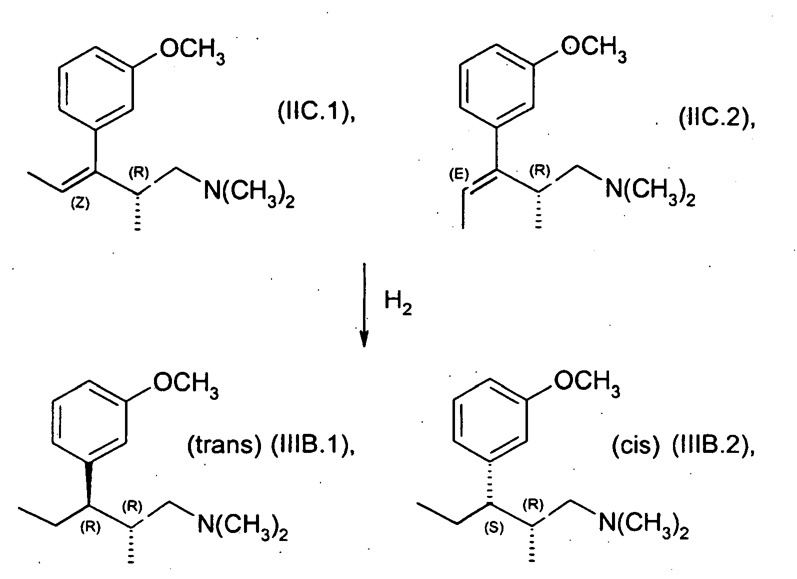

In dem erfindungsgemässen Verfahren wird daher
bevorzugt (Z)-(2R)-3-[(3-Methoxy-phenyl)-2-methyl-pent-3-enyl]-dimethylamin gemäss der Formel IIB.1 eingesetzt, und
bevorzugt überwiegend das Diastereomere (2R, 3R)-[3-(3-Methoxy-phenyl)-2-methyl-pentyl]-dimethyl-amin der Formel IIIB.1 hergestellt.

Ersteres bedeutet, dass das Gemisch der Vorstufe entsprechend dem Fachmann bekannten - nachfolgend kurz beschriebenen - Methoden aufbereitet wird, um das Z-Isomer für die Hydrierung bereitzustellen.

Letzteres bedeutet, dass durch entsprechende Wahl des chiralen Katalysators überwiegend das Diastereomere (2R, 3R)-[3-(3-Methoxy-phenyl)-2-methyl-pentyl]-dimethyl-amin, also die trans-Form, erhalten wird.

Ein hohes Mass an Stereoselektiviät kann dermassen definiert werden, dass die Hydrierung ein Produkt mit einem Enatiomerenüberschuss ("enantiomeric excess", abgekürzt "ee"), respektive Diasteromerenverhältnis ("diastereomeric ratio", abgekürzt "d.r."), ergibt. Der Enatiomerenüberschuss ist definiert als das Verhältnis (%*R* - %*S*) / (%*R* + %*S*), wobei %*R* der prozentuale Anteil der *R*-Form und %*S* der prozentuale Anteil der S-Form an einen Chiralitätszentrum darstellt. Da die Verbindungen der Formeln IIB.1 und IIB.2 am C2-Atom die R-Form aufweisen, kann nach der Hydrierung das nunmehr chirale Zentrum am C3-Atom entweder ebenfalls in der *R*- oder *S*-Form vorliegen. Somit können zwei Diastereomere vorliegen, nämlich die (2*R*, 3*R*)- oder trans-Form und die (2*R*, 3*S*)- oder cis-Form. Nachfolgend wird in der Regel vereinfachend von der trans- oder cis-Form gesprochen, wobei dann die vorgängig angesprochenen jeweiligen Diastereomere gemeint sind.

Mit dem erfindungsgemässen Verfahren kann die Hydrierung ausgehend von einem Gemisch von Verbindungen der Formeln IIC.1 und IIC.2 durchgeführt werden, und trotzdem ein Diastereomerenüberschuss von entweder der trans- oder der cis-Form erhalten werden. Aus Gründen der Verfahrensoptimierung ist es jedoch vorteilhaft von entweder dem isolierten Z-oder dem isolierten E-Isomer auszugehen, da die Isolierung eines der Isomeren zusätzlich der Entfernung von Nebenprodukten ermöglicht.

Im Rahmen der vorliegenden Erfindung wird bevorzugt vom Z-Isomer als Edukt ausgegangen. Durch die Wahl eines entsprechenden Katalysators kann ausgehend vom Z-Isomer ein Verhältnis wahlweise selektiv zugunsten der cis- oder der trans-Form des Produktes erreicht werden. Als gute Selektivität wird dabei ein Verhältnis von grösser oder gleich 70 : 30, bevorzugt grösser oder gleich 75 : 25, besonders bevorzugt grösser oder gleich 80 : 20, insbesondere bevorzugt grösser oder gleich 85 : 15, ganz besonders bevorzugt grösser oder gleich 90 : 10, zugunsten der gewünschten cis- oder trans-Form definiert.

Die Ausgangsverbindungen für das erfindungsgemässe Verfahren in Form der substituierten Dimethyl-(3-aryl-but-3-enyl)-amin-Verbindungen der allgemeinen Formeln II, IIA, IIB und IIC können z.B. durch Dehydratisierung aus substituierten 4-Dimethylamino-2-aryl-butan-2-ol-Verbindungen der allgemeinen Formel I, 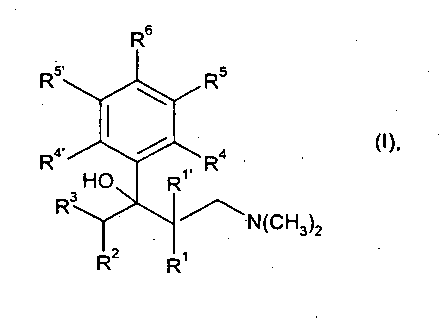 erhalten werden. Die Dehydratisierung kann mittels Säuren erfolgen oder bevorzugt auch gemäss dem Verfahren wie es in der WO 2005/000788 A1 beschrieben ist. Die substituierten Dimethyl-(3-aryl-but-3-enyl)-amin-Verbindungen der allgemeinen Formeln II, IIA, IIB und IIC können in Form eines Gemisches ihrer Stereoisomeren vorliegen. Diese können nach üblichen, dem Fachmann bekannten Methoden, voneinander getrennt werden.

Die Umsetzung der substituierten Dimethyl-(3-aryl-but-3-enyl)-amin-Verbindungen der allgemeinen Formeln 11, IIA, IIB und IIC zu substituierten Dimethyl-(3-aryl-butyl)-amin-Verbindungen der allgemeinen Formel III führt gegebenenenfalls ebenfalls zu einem Gemisch unterschiedlicher Stereoisomere, die nach üblichen, dem Fachmann bekannten Methoden voneinander getrennt werden können. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die substituierten Dimethyl-(3-aryl-but-3-enyl)-amin-Verbindungen der allgemeinen Formeln II, IIA und IIB können in dem erfindungsgemässen Verfahren jeweils sowohl in Form ihrer Basen, ihrer Säuren als auch jeweils in Form ihrer Salze oder jeweils in Form entsprechender Solvate, vorzugsweise Hydrate, eingesetzt werden. Selbstverständlich können auch jeweils Mischungen aus zwei oder mehr der vorstehend genannten Verbindungen zum Einsatz kommen.

Sofern eine substituierte Dimethyl-(3-aryl-but-3-enyl)-amin-Verbindung der allgemeinen Formeln II, IIA, IIB und IIC in Form eines Salzes nach den erfindungsgemässen Verfahren umgesetzt wird, kann dieses bevorzugt ausgewählt werden aus der Gruppe bestehend aus Chlorid, Bromid, Sulfat, Sulfonat, Phosphat, Tartrat, Embonat, Formiat, Acetat, Propionat, Benzoat, Oxalat, Succinat, Citrat, Glutamat, Fumarat, Aspartat, Glutarat, Stearat, Butyrat, Malonat, Lactat, Mesylat, Saccharinat, Cyclamat und, besonders bevorzugt aus der Gruppe Chlorid, Sulfat, Saccharinat, Teoclat und Embonat.

Üblicherweise liegen die Salze dabei in Form eines entsprechenden Säureadditionssalzes, z.B. als Hydrochlorid, vor.

Sofern die substituierten Dlmethyl-(3-aryl-but-3-enyl)-amin-Verbindungen der allgemeinen Formeln II, IIA, IIB und IIC oder die substitulerten Dimethyl-(3-aryl-butyl)amin-Verbindungen der allgemeinen Formel III nach den erfindungsgemässen Verfahren in Form ihrer Basen erhalten werden, können sie nach üblichen, dem Fachmann bekannten Verfahren in die entsprechenden Salze, vorzugsweise in eines der vorstehend aufgeführten Salze, übergeführt werden.

Für das erfindungsgemässe Verfahren der Hydrierung der oben beschrieben Verbindungen der allgemeinen Formeln II, IIA, IIB und IIC mit Wasserstoff mittels homogener Katalyse sind Metallkomplexe des Rhodiums mit Diphosphin-Liganden besonders geeignet.

Über die homogene katalytische von homoallylischen Aminen der allgemeinen Formel II ist wenig bekannt ist. Umso mehr überrascht es, dass man ausgezeichnete Umsätze, Ausbeuten und sehr hohe optische Ausbeuten an gewünschten Stereoisomeren erzielen kann, insbesondere bei Verwendung von Rhodiumkomplexen. Es kann vorteilhaft sein, hierfür von stereochemisch reinen Edukten mit hoher chemischer Reinheit auszugehen. Gegebenenfalls können aber auch nicht aufbereitete Gemische, in denen gleichermassen Z- und E-Isomere vorliegen, eingesetzt werden.

Als Diphosphin-Liganden kommen zum Beispiel Diphosphine und Analoge in Frage, wie sie zum Beispiel in aktuellen Übersichten zu finden sind, unter anderem in a) H. Brunner, W. Zettlmeier, Handbook of Enantioselective Catalysis. VCH Weinheim, 1993, Vol. 2, Seite 3ff.; b) R. Noyori, et al. in Catalytic Asymmetric Synthesis Second Edition (I. Ojima, Ed.), Wiley-VCH, Weinheim, 2000, Seite 1ff.; c) E.N. Jacobsen, A. Pfaltz, H. Yamamoto (Eds.), Comprehensive Asymmetric Catalysis Vol I-III, Springer Berlin, 1999, und darin angegebene Referenzen.

Möglich sind generell achirale und chirale Strukturen der Art Sekundärphosphin-Gerüst-Sekundärphosphin. Die zwei Sekundärphosphingruppen sind bevorzugt so an ein Gerüst gebunden, dass im Metallkomplex zusammen mit dem Metallatom ein 5- bis 10-gliedriger und bevorzugter 5- bis 8-gliedriger Ring gebildet wird. Die zwei Sekundärphosphingruppen sind endständig an den C-Atomen einer C₂-C₈-, bevorzugt C₂-C₆-, und besonders bevorzugt C₂-C₄-Kette gebunden, wobei C-Atome der Kette durch Heteroatome O, S, NH und/oder N-C₁-C₄-Alkyl ersetzt und die Kohlenstoffkette Teil eines monozyklischen oder polyzyklischen Ringes sein können. Das Gerüst kann 2 bis 30, bevorzugt zwei bis 20 C-Atome und gegebenenfalls zusätzlich 2 bis 4 Heteroatome enthalten. Das Gerüst kann unsubstituiert oder substituiert sein, zum Beispiel mit C₁-C₆-Alkyl, C₁-C₆-Alkoxy, C₁-C₆-Alkylthio, C₄-C₈-Cycloalkyl, Phenyl, Benzyl, Phenoxy, Benzyloxy, Phenylthio, Benzylthio, Halogen (bevorzugt F, Cl, Br), OH, Tri(C₁-C₆-Alkyl)silyl, Sekundäramino, -CO₂H, -SO₃H, -CO₂R', -SO₃R'; -O-C(O)-R', -NH-C(O)R', -O-SO₃-R', und -NH-SO₃R', wobei R' für C₁-C₆-Alkyl, C₄-C₈-Cycloalkyl, Phenyl oder Benzyl steht. Bei dem Gerüst kann es sich um bivalente Reste von Alkanen, Heteroalkanen, Alkenen, Zykloalkanen, Zykloalkenen, Heterozykloalkanen, Heterozykloalkenen, Bizykloalkanen, Bizykloheteroalkanen, Spirobiszykloalkanen, Spirobiszykloheteroalkanen, Arylenen, Heteroarylenen, Bisrarylenen, Bisheteroarylenen, Metallocenen wie zum Beispiel Ferrocenen, wobei eine oder beide Phosphingruppen über eine Methylen, C₁-C₁₂-Alkyliden, Phenylen oder -CR"R*-Phenylen an den Cyclopentadienylring eines Metallocens gebunden sein können. R" und R* sind unabhängig voneinander zum Beispiel C₁-C₆-Alkyl, C₁-C₆-Alkoxy, oder Phenyl. Die freien Bindungen befinden sich an einem oder beiden Cyclopentadienylringen. In den zyklischen Gerüsten befinden sich die freien Bindungen bevorzugt in 1,2-Stellungen und in 1,1'-Bisarylen 6,6'-Stellung.

Die Sekundärphosphingruppen können auch über ein Sauerstoffatom an C-Atome des Gerüstes gebunden sein (es handelt sich dann um Phosphinite).

Die Chiralität der Diphosphinliganden kann auf einer planaren Isomerie (Ferrocene), Atropisomerie, der Anwesenheit asymmetrischer C-Atome und/oder P-Atome oder Kombinationen davon beruhen.

Beispiele für Gerüste von Atropisomeren sind 1,1'-Bisaryle und -Bisheteroaryle (Bisaryle wie zum Beispiel Biphenyl, Binaphthyl oder Bisthiophenyl) mit in den 2,2'-Stellungen gebundenen sekundären Phosphingruppen und gegebenenfalls weiteren Substituenten besonders in der 6- oder in den 6,6'-Stellungen. Trivialnamen für solche Liganden sind Binap, Biphemp, Biphep und Solphos. Bekannt sind auch Bicyclopentane als Grundgerüst, die unter dem Trivialnamen Bicp käuflich sind.

Beispiele für Gerüste mit planarer Chiralität sind solche auf Basis von Ferrocenen mit zwei direkt an je einen der Cyclopentadienylringe gebundenen oder an einen Cyclopentadienylring in 1,2-Stellung gebundenen sekundären Phosphingruppen und gegebenenfalls chiralen Substituenten an einem oder beiden Cyclopentadienylringen. Ein anderes Beispiel sind Ferrocene, an die in 1,2-Stellung des Cyclopentadienylringes eine sekundäre Phosphingruppe und eine weitere sekundäre Phosphingruppe über ein asymmetrisches C-Atom gebunden ist. Ein weiteres Beispiel sind Ferrocene, an die in 1,2-Stellung des Cyclopentadienylringes eine sekundäre Phosphingruppe über ein asymmetrisches C-Atom und eine zweite sekundäre Phosphingruppe über 1,2-Phenylen gebunden ist. Trivialnamen für solche Liganden sind Josiphos, Walphos, Taniaphos, Mandyphos und Ferriphos.

Bekannt sind auch Diphosphine mit chiralen P-Ringen, die besonders besonders in einer oder beiden α-Stellungen zum P-Atom substituiert sind, zum Beispiel Phospholane und Phosphetane. Solche sekundäre Phoshingruppen können in 1,2-Stellung von Benzol, Naphthalin, Thiophen, Benzothiophen, Ethan und Ferrocen gebunden sein. Bekannte Trivialnamen sind Rophos, Butiphane und Kephos.

Beispiele für Gerüste mit asymmetrischen C-Atomen sind offenkettige mit in 1,2-, 1,3- oder 1,4-Stellungen, aliphatische bizyklische Ringsysteme mit in den 1,2-Stellungen gebundenen sekundären Phosphingruppen, oder zyklische oder heterozyklische Fünfringe mit in 3,4-Stellungen gegebenenfalls über eine Methylengruppe gebundenen sekundären Phosphingruppen. Bekannt sind auch Fünfringe mit in 4-Stellung gebundener sekundärer Phosphingruppe und in 2-Stellung gebundener sekundärer Phosphinmethylgruppe. Trivialnamen für solche Liganden sind Diop, Bppm, Bzppm, Depyphos, Norphos und Prophos.

Beispiele für Diphosphine mit chiralen P-Atomen sind 1,2-Bis(sekundäphosphin)ethane mit unterschiedlichen Substituenten in den Phosphingruppen. Ein bekannter Vertreter ist unter dem Trivialnamen Dipamp erhältlich.

Die sekundären Phosphingruppen können gleiche oder verschiedene Kohlenwasserstoffreste als Substituenten enthalten und die beiden sekundären Phosphingruppen in den Diphosphinen können gleich oder verschieden sein. Häufig können gute Ergebnisse erzielt werden, wenn die sekundären Phosphingruppen nicht identisch, sondern verschieden sind.

Die Kohlenwasserstoffreste können unsubstituiert oder substituiert sein und und/oder Heteroatome ausgewählt aus der Gruppe O, S, -N= oder N(C₁-C₄-Alkyl) enthalten. Sie können 1 bis 22, bevorzugt 1 bis 12, und besonders bevorzugt 1 bis 8 C-Atome enthalten.

Ein bevorzugtes Sekundärphosphin ist jenes, worin die Phosphingruppe zwei gleiche oder verschiedene Reste, ausgewählt aus der Gruppe lineares oder verzweigtes C₁-C₁₂-Alkyl; unsubstituiertes oder mit C₁-C₆-Alkyl oder C₁-C₆-Alkoxy substituiertes C₅-C₁₂-Cycloalkyl oder C₅-C₁₂-Cycloalkyl-CH₂-; Phenyl, Naphthyl, Furyl oder Benzyl; oder mit Halogen, C₁-C₆-Alkyl, Trifluormethyl, C₁-C₆-Alkoxy, Trifluormethoxy, (C₆H₅)₃Si, (C₁-C₁₂-Alkyl)₃Si, oder Sekundäramino substituiertes Phenyl oder Benzyl, enthält.

Beispiele für P-Substituenten als Alkyl, das bevorzugt 1 bis 6 C-Atome enthält, sind Methyl, Ethyl, n-Propyl, i-Propyl, n-Butyl, i-Butyl, t-Butyl, und die Isomeren von Pentyl und Hexyl. Beispiele für P-Substituenten als gegebenenfalls mit Alkyl substituiertes Cycloalkyl sind Cyclopentyl, Cyclohexyl, Methyl- und Ethylcyclohexyl, und Dimethylcyclohexyl. Beispiele für P-Substituenten als mit Alkyl, und Alkoxy substituiertes Phenyl und Benzyl sind Methylphenyl, Dimethylphenyl, Trimethylphenyl, Ethylphenyl, Methylbenzyl, Methoxyphenyl, Dimethoxyphenyl, Trimethoxyphenyl, Trifluormethylphenyl, Bis-trifluormethylphenyl, Tris-trifluormethylphenyl, Trifluormethoxyphenyl, Bis-trifluormethoxyphenyl, Fluor- und Chlorphenyl und 3,5-Dimethyl-4-methoxyphenyl.

Bevorzugte sekundäre Phosphingruppen sind solche, die gleiche oder verschiedene Reste ausgewählt aus der Gruppe C₁-C₆-Alkyl, unsubstituiertes oder mit 1 bis 3 C₁-C₄-Alkyl oder C₁-C₄-Alkoxy substituiertes Cyclopentyl oder Cyclohexyl, Benzyl und besonders Phenyl, die unsubstituiert oder substituiert sind mit 1 bis 3 C₁-C₄-Alkyl, C₁-C₄-Alkoxy, C₁-C₄-Fluoralkyl oder C₁-C₄-Fluoralkoxy, F und Cl.

Die Sekundärphosphinogruppe entspricht bevorzugt der Formel -PR₁₆R₁₇, worin R₁₆ und R₁₇ unabhängig voneinander einen Kohlenwasserstoffrest mit 1 bis 18 C-Atomen darstellen, der unsubstituiert oder substituiert ist mit C₁-C₆-Alkyl, Trifluormethyl, C₁-C₆-Alkoxy, Trifluormethoxy, (C₁-C₄-Alkyl)₂amino, (C₆H₅)₃Si, (C₁-C₁₂-Alkyl)₃Si, Halogen, und/oder Heteroatome O enthält.

Bevorzugt sind R₁₆ und R₁₇ Reste, ausgewählt aus der Gruppe lineares oder verzweigtes C₁-C₆-Alkyl, unsubstituiertes oder mit ein bis drei C₁-C₄-Alkyl oder C₁-C₄-Alkoxy substituiertes Cyclopentyl oder Cyclohexyl, Furyl, unsubstituiertes oder mit ein bis drei C₁-C₄-Alkyl oder C₁-C₄-Alkoxy substituiertes Benzyl, und insbesondere unsubstituiertes oder mit ein bis drei F, Cl, C₁-C₄-Alkyl, C₁-C₄-Alkoxy, C₁-C₄-Fluoralkyl oder C₁-C₄-Fluoralkoxy substituiertes Phenyl.

Besonders bevorzugt bedeuten R₁₆ und R₁₇ Reste, ausgewählt aus der Gruppe C₁-C₆-Alkyl, Cyclopentyl, Cyclohexyl, Furyl, und unsubstituiertes oder mit ein bis drei F, Cl, C₁-C₄-Alkyl, C₁-C₄-Alkoxy und/oder C₁-C₄-Fluoralkyl substituiertes Phenyl.

Wenn R₁₆ und R₁₇ in der Gruppe -PR₁₆R₁₇ verschieden sind, dann liegen Liganden vor, die zusätzlich P-chiral sind.

Bei der sekundären Phosphingruppe kann es sich um zyklisches Sekundärphosphino handeln, zum Beispiel solche der Formeln 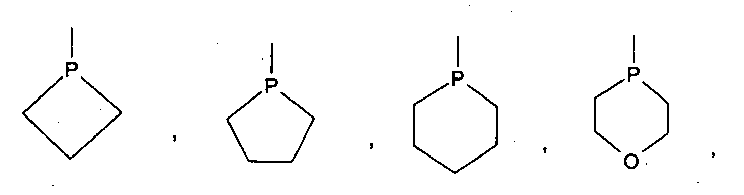 die unsubstituiert oder ein- oder mehrfach substituiert sind mit C₁-C₈-Alkyl, C₄-C₈-Cycloalkyl, C₁-C₆-Alkoxy, C₁-C₄-Alkoxy-C₁-C₄-Alkyl, Phenyl, C₁-C₄-Alkyl- oder C₁-C₄-Alkoxyphenyl, Benzyl, C₁-C₄-Alkyl- oder C₁-C₄-Alkoxybenzyl, Benzyloxy, C₁-C₄-Alkyl- oder C₁-C₄-Alkoxybenzyl-oxy, oder C₁-C₄-Alkyliden-dioxyl.

Die Substituenten können in einer oder beiden α-Stellungen zum P-Atom gebunden sein, um chirale C-Atome einzuführen. Bei den Substituenten in einer oder beiden α-Stellungen handelt es sich bevorzugt um C₁-C₄-Alkyl oder Benzyl, zum Beispiel um Methyl, Ethyl, n- oder i-Propyl, Benzyl oder -CH₂-O-C₁-C₄-Alkyl beziehungsweise -CH₂-O-C₆-C₁₀-Aryl.

Bei Substituenten in den β,γ-Stellungen kann es sich zum Beispiel um C₁-C₄-Alkyl, C₁-C₄-Alkoxy, Benzyloxy, oder -O-CH₂-O-, -O-CH(C₁-C₄-Alkyl)-O-, und -O-C(C₁-C₄-Alkyl)₂-O- handeln. Einige Beispiele sind Methyl, Ethyl, Methoxy, Ethoxy, -O-CH(Methyl)-O-, und -O-C(Methyl)₂-O-.

Je nach Art der Substitution und Anzahl der Substituenten können die zyklischen Phosphinreste C-chiral, P-chiral oder C- und P-chiral sein.

An zwei benachbarte C-Atome in den Resten der obigen Formeln kann ein aliphatischer 5- oder 6-Ring oder Benzol ankondensiert sein.

Das zyklische Sekundärphosphino kann zum Beispiel den nachfolgenden Formeln (nur eines der möglichen Diastereomeren ist angegeben) entsprechen, 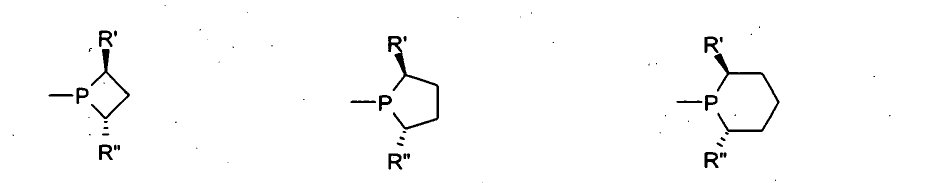 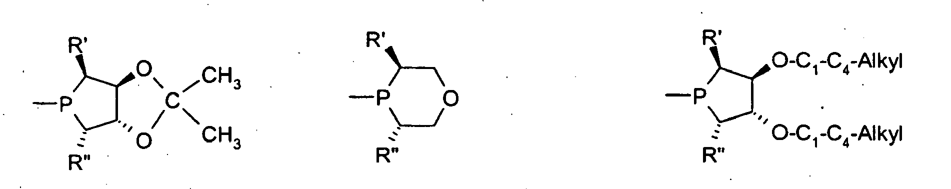 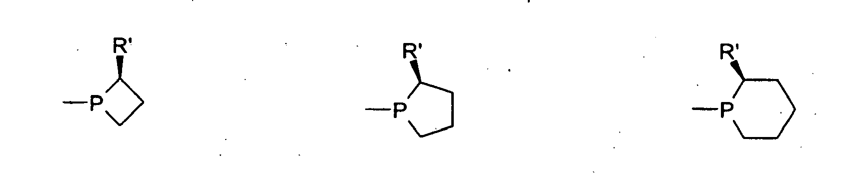 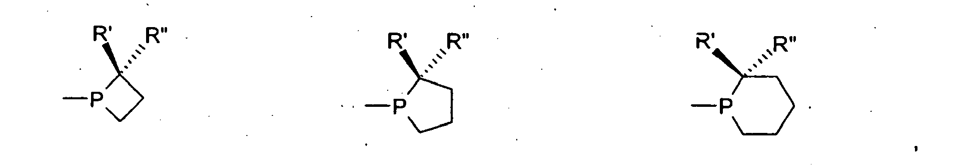 worin
die Reste R' und R" für C₁-C₄-Alkyl, zum Beispiel Methyl, Ethyl, n- oder i-Propyl, Benzyl, oder -CH₂-O-C₁-C₄-Alkyl beziehungsweise -CH₂-O-C₆-C₁₀-Aryl stehen, und R' und R" gleich oder voneinander verschieden sind.

Die zwei Sekundärphosphinoreste -PR₁₆R₁₇ in Diphosphinen bedeuten bevorzugt unabhängig voneinander nicht-zyklisches Sekundärphosphin ausgewählt aus der Gruppe -P(C₁-C₆-Alkyl)₂, -P(C₅-C₈-Cycloalkyl)₂, -P(C₇-C₈-Bicycloalkyl)₂, -P(o-Furyl)₂, ₋P(C₆H₅)₂, -P[2-(C₁-C₆-Alkyl)C₆H₄]₂, -P[3-(C₁-C₆-Alkyl)C₆H₄]₂, -P[4-(C₁-C₆-Alkyl)C₆H₄]₂, -P[2-(C₁-C₆-Alkoxy)C₆H₄]₂, -P[3-(C₁-C₆-Alkoxy)C₆H₄]₂, -P[4-(C₁-C₆-Alkoxy)C₆H₄]₂, -P[2-(Trifluormethyl)C₆H₄]₂, -P[3-(Trifluormethyl)C₆H₄]₂, -P[4-(Trifluormethyl)C₆H₄]₂, -P[3,5-Bis(trifluormethyl)C₆H₃]₂, -P[3,5-Bis(C₁-C₆-Alkyl)₂C₆H₃]₂, -P[3,5-Bis(C₁-C₆-Alkoxy)₂C₆H₃]₂, und -P[3,5-Bis(C₁-C₆-Alkyl)₂-4-(C₁-C₆-Alkoxy)C₆H₂]₂, oder zyklisches Phosphin, ausgewählt aus der Gruppe 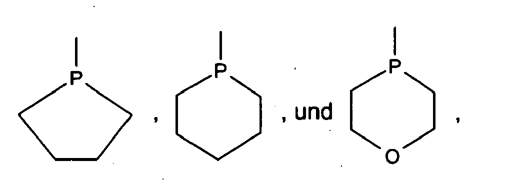 die unsubstituiert oder ein- oder mehrfach substituiert sind mit C₁-C₄-Alkyl, C₁-C₄-Alkoxy, C₁-C₄-Alkoxy-C₁-C₂-Alkyl, Phenyl, Benzyl, BenzyloXy, oder C₁-C₄-Alkyliden-dioxyl.

Einige spezifische Beispiele sind -P(CH₃)₂, -P(i-C₃H₇)₂, -P(n-C₄H₉)₂, -P(i-C₄H₉)₂, -P(t-C₄H₉)₂, -P(C₅H₉), -P(C₆H₁₁)₂, -P(Norbornyl)_{2,} -P(o-Furyl)₂, -P(C₆H₅)₂, P[2-(Methyl)C₆H₄]₂, P[3-(Methyl)C₆H₄]₂, -P[4-(Methyl)C₆H₄]₂, -P[2-(Methoxy)C₆H₄]₂, -P[3-(Methoxy)C₆H₄)₂, -P[4-(Methoxy)C₆H₄]₂, -P[3-(Trifluormethyl)C₆H₄]₂, -P[4-(Trifluormethyl)C₆H₄]₂, -P[3,5-Bis(trifluormethyl)-C₆H₃]₂, -P[3,5-Bis(methyl)₂C₆H₃]₂, -P[3,5-Bis(methoxy)₂C₆H₃]₂, und -P[3,5-Bis(methyl)₂-4-(methoxy)C₆H₂]₂, und solche der Formeln 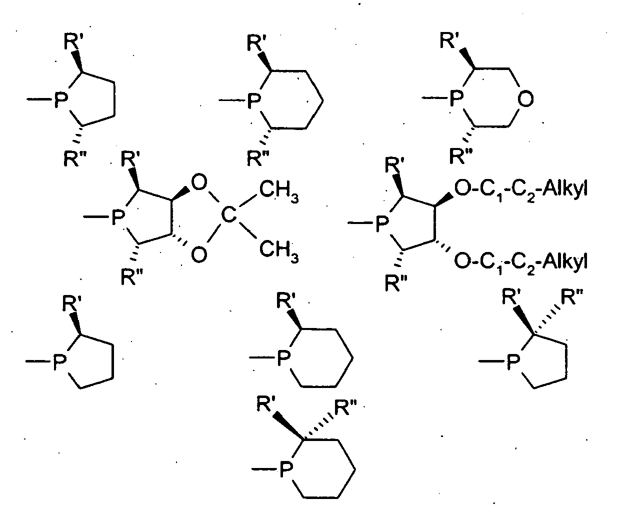 worin
R' Methyl, Ethyl, Methoxy, Ethoxy, Phenoxy, Benzyloxy, Methoxy-methyl, Ethoxymethyl oder Benzyloxymethyl darstellt und R" unabhängig die gleiche Bedeutung wie R' hat.

Bevorzugte Diphosphinliganden sind ausgewählt aus solchen der Formeln:
A) Liganden der allgemeinen Formel: 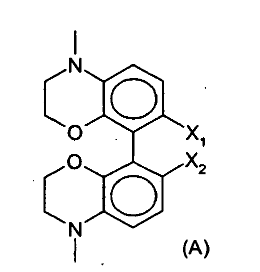
(B) Liganden der allgemeinen Formel: 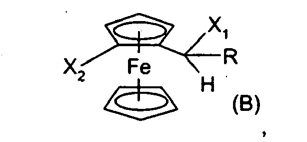
(C) Liganden der allgemeinen Formel: 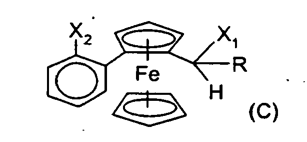
(D-G) Liganden der allgemeinen Formeln:

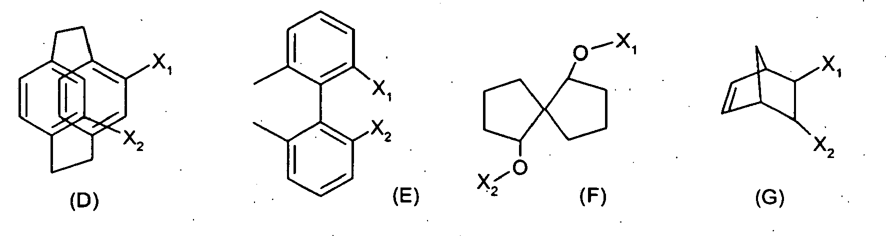

In den Liganden der allgemeinen Formeln A bis G bedeuten X₁ und X₂ P-gebundene sekundäre Phosphingruppen, einschliesslich der Ausgestaltungen und Bevorzugungen.

Ein ganz besonders bevorzugter Ligand der allgemeinen Formel A weist als Rest X₁ und X₂ jeweils eine unsubstituierte Diphenylphosphingruppe auf.

Ein ganz besonders bevorzugter Ligand der allgemeinen Formel B weist als Rest X₁ eine Di-(2-Furyl)phosphingruppe, als Rest X₂ eine Dixylylphosphingruppe, und als Rest R eine Methylgruppe auf.

Ein ganz besonders bevorzugter Ligand der allgemeinen Formel C weist als Rest X₁ eine Dixylylphosphingruppe, als Rest X₂ ebenfalls eine Dixylylphosphingruppe, und als Rest R eine Methylgruppe auf.

Ganz besonders bevorzugte Liganden der allgemeinen Formeln D, F und G weisen als Rest X₁ und X₂ jeweils eine unsubstituierte Diphenylphosphingruppe auf.

Ein ganz besonders bevorzugter Ligand der allgemeinen Formel E weist als Rest X₁ eine Dixylylphosphingruppe und als Rest X₂ eine Dicyclohexylphosphingruppe auf.

R in den allgemeinen Formeln B und C steht für die möglichen Reste C₁-C₄-Alkyl, bevorzugt Methyl, C₆-C₁₀-Aryl, bevorzugt Phenyl, oder C₇-C₁₂-Aralkyl, bevorzugt Benzyl.

Weiterhin bevorzugte Liganden sind solche der allgemeinen Formeln H und I: 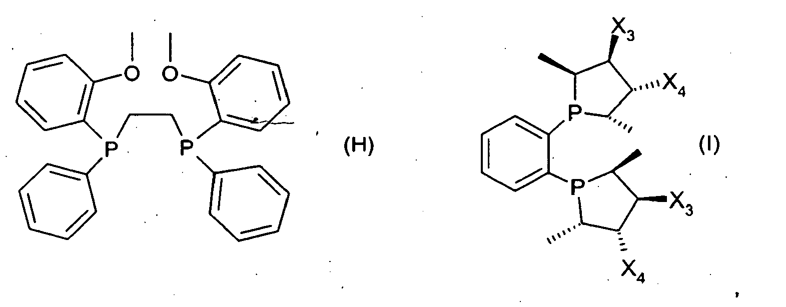 worin X₃ und X₄ unabhängig voneinander H, OH, C₁-C₄-Alkyl, C₁-C₄-Alkoxy, Benzyloxy, - CH(C₁-C₄-Alkyl)- oder -CH(Phenyl)-, vorzugsweise je -OH bedeuten.

Schliesslich sind bevorzugte Diphosphin-Liganden generell solche, mit denen sich ein Diastereomeren Verhältnis zugunsten entweder der trans- oder der cis-Form von grösser oder gleich 70 : 30, bevorzugt grösser oder gleich 75 : 25, besonders bevorzugt grösser oder gleich 80 : 20, insbesondere bevorzugt grösser oder gleich 85 : 15, ganz besonders bevorzugt grösser oder gleich 90 : 10 erreichen lässt.

Ganz besonders bevorzugte Ausführungsformen des erfindungsgemässen Verfahrens sind:
a) Verfahren, dadurch gekennzeichnet, dass man eine Verbindung der Formel IIC.1 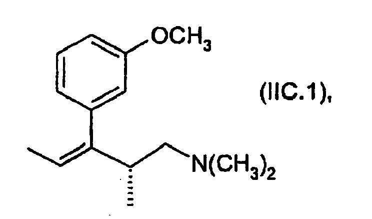 einsetzt, der Diphosphinligand ausgewählt ist aus einer Verbindung der Formeln A bis F, und als Verbindung der Formel III überwiegend das Diastereomere (2R,3R)-[3-(3-Methoxy-phenyl)-2-methyl-pentyl]-dimethyl-amin erhalten wird, wobei das Diastereomerenverhältnis (2R,3R):(2S,3R) bei grösser oder gleich 75 : 25 liegt.
b) Verfahren, dadurch gekennzeichnet, dass man eine Verbindung der Formel IIC.1 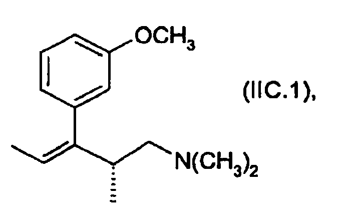 einsetzt, der Diphosphinligand ausgewählt ist aus einer Verbindung der Formeln G, H oder I gemäss Anspruch 11, und als Verbindung der Formel III überwiegend das Diastereomere (2*S*,3*R*)-[3-(3-Methoxy-phenyl)-2-methyl-penty]-dimethyl-amin erhalten wird, wobei das Diastereomerenverhältnis (2S,3R):(2R,3R) bei grösser oder gleich 75 : 25 liegt.

Die disekundären Diphosphine, einschliesslich der Ausgestaltungen und Bevorzugungen, sind Liganden für Metallkomplexe des Rhodiums, die hervorragende Katalysatoren oder Katalysatorvorläufer für die asymmetrische Hydrierung der prochiralen oder chiralen, ungesättigten, organischen Verbindungen der allgemeinen Formel II darstellen.

Die Metallkomplexe können je nach Oxidationszahl und Koordinationszahl des Rhodiums weitere Liganden und/oder Anionen enthalten. Es kann sich auch um kationische Metallkomplexe handeln. Solche analogen Metallkomplexe und deren Herstellung sind vielfach in der Literatur beschrieben.

Die Metallkomplexe können zum Beispiel den allgemeinen Formeln IV und V entsprechen,

A₁MeLₙ (IV),

(A₁MeLₙ)^{(z+)}(E⁻)_{z} (V),

worin A₁ für einen Diphosphin-Liganden, einschliesslich der Ausgestaltungen und Bevorzugungen, insbesondere der Formeln A bis I, steht,
L für gleiche oder verschiedene monodentate, anionische oder nicht-ionische Liganden steht, oder zwei L für gleiche oder verschiedene bidentate, anionische oder nicht-ionische Liganden steht;
n für 2, 3 oder 4 steht, wenn L einen monodentaten Liganden bedeutet, oder n für 1 oder 2 steht, wenn L einen bldentaten Liganden bedeutet;
z für 1, 2 oder 3 steht;
Me = Rhodium (Rh) bedeutet; wobei das Metall die Oxidationsstufen 0, 1, 2, 3 oder 4 aufweist;
E das Anion einer Sauerstoffsäure oder Komplexsäure ist; und
die anionischen Liganden die Ladung der Oxidationsstufen 1, 2, 3 oder 4 des Metalls ausgleichen.

Monodentate nicht-ionische Liganden können zum Beispiel ausgewählt sein aus der Gruppe der Olefine (zum Beispiel Ethylen, Propylen), Allyle (Allyl, 2-Methallyl), solvatisierenden Lösungsmitteln (Nitrile, lineare oder cyclische Ether, gegebenenfalls N-alkylierte Amide und Lactame, Amine, Phosphine, Alkohole, Carbonsäureester, Sulfonsäurester), Stickstoffmonoxid und Kohlenmonoxid.

Monodentate anionische Liganden können zum Beispiel ausgewählt sein aus der Gruppe Halogenid (F, Cl, Br, I), Pseudohlogenid (Cyanid, Cyanat, Isocyanat) und Anionen von Carbonsäuren, Sulfonsäuren und Phosphonsäuren (Carbonat, Formiat, Acetat, Propionat, Methylsulfonat, Trifluormethylsulfonat, Phenylsulfonat, Tosylat).

Bidentate nicht-ionische Liganden können zum Beispiel ausgewählt sein aus der Gruppe der linearen oder cyclischen Diolefine (zum Beispiel Hexadien, Cyclooctadien, Norbornadien), Dinitrile (Malondinitril), gegebenenfalls N-alkylierte Carbonsäurediamide, Diaminen, Diphosphinen, Diolen, Acetonylacetonate, Dicarbonsäurediester und Disulfonsäurediester.

Bidentate anionische Liganden können zum Beispiel ausgewählt sein aus der Gruppe der Anionen von Dicarbonsäuren, Disulfonsäuren und Diphosphonsäuren (zum Beispiel von Oxalsäure, Malonsäure, Bernsteinsäure, Maleinsäure, Methylendisulfonsäure und Methylendiphosphonsäure).

Bevorzugte Metallkomplexe sind auch solche, worin E für -Cl⁻, -Br⁻, -I⁻, ClO₄⁻, CF₃SO₃⁻, CH₃SO₃⁻, HSO₄⁻, BF₄⁻, B(Phenyl)₄⁻, B(C₆F₅)₄⁻, B(3,5-Bistrifluormethyl-phenyl)₄⁻, PF_{B}⁻, SbCl₆⁻, AsF₈⁻ oder SbF₆⁻ steht.

Insbesondere bevorzugte Metallkomplexe entsprechen den Formeln VI und VII,

[A₁Me₁YZ] (VI),

[A₁Me₁Y]⁺E₁⁻ (VII),

worin
A₁ für einen Diphosphin-Liganden, einschliesslich der Ausgestaltungen und Bevorzugungen, insbesondere der Formeln A bis I, steht;
Me₁ Rhodium (Rh) bedeutet;
Y für zwei Olefine oder ein Dien steht;
Z Cl, Br oder I bedeutet; und
E₁⁻ das Anion einer Sauerstoffsäure oder Komplexsäure darstellt.

Bei Y in der Bedeutung als Olefin kann es sich um C₂-C₁₂-, bevorzugt C₂-C₆- und besonders bevorzugt C₂-C₄-Olefine handeln. Beispiele sind Propen, But-1-en und besonders Ethylen. Das Dien kann 5 bis 12 und bevorzugt 5 bis 8 C-Atome enthalten und es kann sich um offenkettige, cyclische oder polycyclische Diene handeln. Die beiden Olefingruppen des Diens sind bevorzugt durch ein oder zwei CH₂-Gruppen verbunden. Beispiele sind 1,3-Pentadien, Cyclopentadien, 1,5-Hexadien, 1,4-Cyclohexadien, 1,4-oder 1,5-Heptadien, 1,4-oder 1,5-Cycloheptadien, 1,4- oder 1,5-Octadien, 1,4- oder 1,5-Cyclooctadien und Norbornadien.

Bevorzugt stellt Y zwei Ethylen oder 1,5-Hexadien, 1,5-Cyclooctadien oder Norbomadien dar.

In Formel VI steht Z bevorzugt für Cl oder Br. Beispiele für E₁ sind ClO₄⁻, CF₃SO₃⁻, CH₃SO₃⁻, HSO₄⁻, BF₄⁻, B(Phenyl)₄⁻, PF_{B}⁻, SbCl₆⁻, AsF₆⁻ oder SbF₆⁻.

Komplexe dieser Art sind in der nachfolgend erwähnten Literatur und der darin zitierten Literatur beschrieben:
D. J. Ager, S. A. Laneman, Tetrahedron: Asymmetry, 8, 1997, 3327 - 3355;
T. Ohkuma, R. Noyori in Comprehensive Asymmetric Catalysis (E.N. Jacobsen, A. Pfaltz, H. Yamamoto, Eds.), Springer, Berlin, 1999, 199-246;
J. M. Brown in Comprehensive Asymmetric Catalysis (E.N. Jacobsen, A. Pfaltz, H. Yamamoto, Eds.), Springer, Berlin, 1999, 122-182;
T. Ohkuma, M. Kitamura, R. Noyori in Catalytic Asymmetric Synthesis, 2nd Edition (I. Ojima, Ed.), Wiley-VCH New York, 2000, 1-110;
N. Zanetti, et al. Organometallics 15, 1996, 860.

Die Metallkomplexe werden nach in der Literatur bekannten Methoden hergestellt (siehe z.B. Comprehensive Asymmetric Catalysis I bis III, Springer Verlag, Berlin, 1999, und darin zitierte Literatur).

Das erfindungsgemässe Verfahren kann hinsichtlich der Verfahrensparameter für die Hydrierung, also unter den Bedingungen hinsichtlich Druck, Temperatur, Lösungsmittel und Mengen, gemäss dem aus den Stand der Technik gebräuchlichen Bedingungen durchgeführt werden.

Die wichtigsten Parameter sind wie folgt zusammengefasst:

Das erfindungsgemässe Verfahren kann bei tiefen oder erhöhten Temperaturen, zum Beispiel Temperaturen von -20 bis 150 °C, bevorzugt von -10 bis 100 °C, und besonders bevorzugt von 10 bis 80 °C durchgeführt werden. Die optischen Ausbeuten sind im allgemeinen bei tieferer Temperatur besser als bei höheren Temperaturen.

Das erfindungsgemässe Verfahren kann bei Normaldruck oder Überdruck durchgeführt werden. Der Druck kann zum Beispiel von 10⁵ bis 2×10⁷ Pa (Pascal) betragen. Hydrierungen werden bevorzugt bei Überdruck durchgeführt.

Katalysatoren werden bevorzugt in Mengen von 0,00001 bis 10 Mol-%, besonders bevorzugt 0,0001 bis 5 Mol-%, und insbesondere bevorzugt 0,001 bis 2 Mol-% verwendet, bezogen auf die zu hydrierende Verbindung.

Die Herstellung der Liganden und Katalysatoren sowie die Hydrierung kann ohne oder in Gegenwart eines inerten Lösungsmittels durchgeführt werden, wobei ein Lösungsmittel oder Gemische von Lösungsmitteln eingesetzt werden können. Geeignete Lösungsmittel sind zum Beispiel aliphatische, cycloaliphatiche und aromatische Kohlenwasserstoffe (Pentan, Hexan, Petrolether, Cyclohexan, Methylcyclohexan, Benzol, Toluol, Xylol), aliphatische Halogenkohlenwasserstoffe (Methylenchlorid, Chloroform, Di- und Tetrachlorethan), Nitrile (Acetonitril, Propionitril, Benzonitril), Ether (Diethylether, Dibutylether, t-Butylmethylether, Ethylenglykoldimethylether, Ethylenglykoldiethylether, Diethylenglykoldimethylether, Tetrahydrofuran, Dioxan, Diethylenglykolmonomethyl- oder monoethyether), Ketone (Aceton, Methylisobutylketon), Carbonsäureester und Lactone (Essigsäureethyl- oder -methylester, Valerolacton), N-substituierte Lactame (N-Methylpyrrolidon), Carbonsäureamide (Dimethylacetamid, Dimethylformamid), acyclische Harnstoffe (Dimethylimidazolin), und Sulfoxide und Sulfone (Dimethylsulfoxid, Dimethylsulfon, Tetramethylensulfoxid, Tetramethylensulfon) und Alkohole (Methanol, Ethanol, Propanol, Butanol, Ethylenglykolmonomethylether, Ethylenglykolmonoethylether, Diethylenglykolmonomethylether, Trifluorethanol) und Wasser. Die Lösungsmittel können alleine oder in Mischung von wenigstens zwei Lösungsmitteln verwendet werden.

Es kann vorteilhaft sein, die Reaktion in Gegenwart von im Reaktionsgemisch löslichen Metall- beziehungsweise Ammoniumhalogeniden durchzuführen, zum Beispiel Alkalimetallchloriden, -bromiden und-iodiden, oder quaternären Ammoniumhalogeniden wie zum Beispiel Tetrabutylammoniumiodid.

Besonders zweckmässig kann es sein, zum Beispiel wenn das Substrat als freie Base eingesetzt wird, die Hydrierung in Gegenwart von Säuren durchzuführen, zum Beispiel organischen Säuren wie Sulfonsäuren (Methansulfonsäure, Trifluormethansäure), Carbonsäuren (Ameisensäure, Essigsäure, Oxalsäure), Phosphonsäuren (Methanphosphonsäure), Mineralsäuren Halogenwasserstoffsäuren (HCl, HBr, HI), Schwefelsäure, Phosphorige Säure, Phosphorsäure (siehe zum Beispiel US-A-5,371,256, US-A-5,446,844 und US-A-5,583,241 und EP-A-0 691 949). Die Säure kann so gewählt werden, dass man direkt ein gewünschtes Salz des Wirkstoffs erhält. Entsprechend kann die Menge an Säure bis zu 1 Äquivalent oder mehr betragen, zum Beispiel ein Überschuss bis zu 1,5 Äquivalente, bezogen auf die Menge des zu hydrierenden Substarts. Ein geeigneter Mengenbereich ist 0,01 bis 1 Äquivalente Säure, bezogen auf die Menge des zu hydrierenden Substrats.

Die als Katalysatoren verwendeten Metallkomplexe können als getrennt hergestellte isolierte Verbindungen zugegeben werden, oder bevorzugt auch in situ vor der Reaktion gebildet und dann mit dem zu hydrierenden Substrat vermischt werden. Es kann vorteilhaft sein, bei der Reaktion unter Verwendung von isolierten Metallkomplexen zusätzlich Liganden zuzugeben, oder bei der in situ Herstellung einen Überschuss der Liganden einzusetzen. Der Überschuss kann zum Beispiel 1 bis 10 und vorzugsweise 1 bis 5 Molprozent betragen, bezogen auf die zur Herstellung verwendete Metallverbindung.

Das erfindungsgemässe Verfahren wird im allgemeinen so durchgeführt, dass man den Katalysator vorlegt und dann das Substrat, gegebenenfalls Reaktionshilfsmittel und die gasförmige anzulagernde Verbindung in Form von Wasserstoff vorzugsweise aufpresst. Das Verfahren kann in verschiedenen Reaktortypen kontinuierlich oder satzweise durchgeführt werden.

Die erfindungsgemäss herstellbaren chiralen organischen Verbindungen sind aktive Substanzen oder Zwischenprodukte zur Herstellung solcher Substanzen, insbesondere im Bereich der Herstellung von Pharmazeutika.

Die nachfolgenden Beispiele erläutern die Erfindung.

### Gaschromatische (GC) Bestimmungen werden folgendermassen durchgeführt:

a) Probenvorbereitung: Zu dem Probenmaterial wird tert-Butyl-methylether hinzugegeben. Hydrochloride werden mit Dowex MWA-1 zur Base freigesetzt. Die klare organische Phase wird injiziert.
b) Gaschromatische Bedingungen: Kapillarsäure: 6% Cyanoproyl-phenyl, 94% dimethylpolysiloxan, z.B. Optima 1301-DF 30 m x 0,32 mm, 1,0 µm Filmdicke; Trägergas: Helium; Vordruck: 70 kPa; Split: 20 ml/min; Ofentemperaturprogramm: Initial 160°C/5 min; Rate 5°C / min; 190°C/9 min; Rate 10°/ min.; 250 °C/ 14 min; Detektor/Temperatur: FID/260°C

### A) Herstellung von Ausgangsverbindungen [verbindungen der allgemeinen Formel (II)]:

### Beispiel A1:

### (Z,E)-(2R)-[3-(3-Methoxy-phenyl)-2-methyl-pent-3-enyl]-dimethyl-amin

In einem 250 ml Dreihalskolben mit Thermometer, mechanischem Druckluftrührer, Rückflusskühler und Ölbadheizung werden 28,7g (0,1mol) (2*S*,3*R*)-1-Dimethylamino-3-(3-methoxy-phenyl)-2-methyl-pentan-3-ol hydrochlorid vorgelegt und mit 150 ml wässriger 36 Gew.-%-iger Salzsäure versetzt. Es wird 1 h Stunde auf 100°C erwärmt. Die Mischung wird auf 20°C abgekühlt und bei 20°C unter Kühlung mit 33 Gew.%-iger Natronlauge einen pH-Wert von 11 eingestellt. Es wird 150ml Essigsäureethylester zugesetzt, 10 min gerührt, der Rührer abgestellt, die Phasen getrennt und der Essigsäureethylester am Rotationsverdampfer bei 60°C bis zu einem Druck von 10 mbar abdestilliert. Der ölige Rückstand besteht aus (*Z,E*)-(2*R*)-[3-(3-Methoxy-phenyl)-2-methyl-pent-3-enyl]-dimethyl-amin mit einer GC-Reinheit von 86%, einem *Z*/*E*-Verhältnis von.6,5:1 und einer Ausbeute von 21g (90% der Theorie). Bei der Reinheitsuntersuchung wurde kein Ausgangsprodukt und 8,5% des (*Z,E*)-[3-(3-Methoxyphenyl)-2-methyl-pent-2-enyl]-dimethyl-amins gefunden.

### Beispiel A2:

### (Z)-(2R)-[3-(3-Methoxy-phenyl)-2-methyl-pent-3-enyl]-dimethyl-amin

201 g (0,86 mol) einer Mischung aus (*Z*)-(2*R*)-[3-(3-Methoxy-phenyl)-2-methyl-pent-3-enyl]-dimethyl-amin, (*E*)-(2*R*)-[3-(3-Methoxy-phenyl)-2-methyl-pent-3-enyl]-dimethyl-amin und Nebenprodukten (siehe Beispiel 1) wird in 1 I Aceton gelöst und mit 15,5 g (0,86 mol) Wasser versetzt. Anschließend wird 94,0 g (0,87 mol) Trimethylchlorsilan zugetropft und bei 5°C - 8°C für 72 h gerührt. Die ausgefallenen Kristalle werden abgesaugt und mit Aceton nachgewaschen. Anschliessend wird das Produkt im Trockenschrank bei 80-120 mbar und 40 - 50° C getrocknet. Das erhaltene (*Z*)-(2*R*)-[3-(3-Methoxy-phenyl)-2-methyl-pent-3-enyl]-dimethyl-amin hydrochlorid-Rohprodukt wird in 513 ml Wasser gelöst und mit Natronlauge ein pH von 11-12 eingestellt. Die wässrige Lösung wird mit 500 ml Ethylacetat versetzt und das Produkt extrahiert. Die abgetrennte Ethylester-Phase wird über Natriumsulfat getrocknet und am Rotationsverdampfer eingeengt. Das so erhaltene (*Z*)-(2*R*)-[3-(3-Methoxy-phenyl)-2-methylpent-3-enyl]-dimethyl-amin wird in 733 ml Aceton gelöst und mit 11,2 g (0,63 mol) Wasser versetzt. Es wird langsam 67,8 g (0,63 mol) Trimethylchlorsilan zugegeben. Das Reaktionsgemisch wird bei 5-8° C für 72 Stunden gerührt. Anschliessend wird der ausgefallene Feststoff abgesaugt und mit Aceton nachgewaschen. Das Produkt wird im Trockenschrank bei 80-120 mbar und 45-50° C für 16 Stunden getrocknet.

Die Ausbeute beträgt 134,4 g (58 %) mit einer Reinheit von 100,0 %. Das Verhältnis von *Z-*zu *E*-Isomer beträgt 99,05 : 0,95.

### B) Hydrierungen (Herstellung der beiden Diastereomere (2R, 3R)-[3-(3-Methoxy-phenyl)-2-methyl-pentyl]-dimethyl-amin und (2R, 3S)-[3-(3-Methoxy-phenyl)-2-methyl-pentyl]-dimethylamin)

### Beispiel B1: Allgemeines Verfahren zur katalytischen Hydrierung:

Ein Autoklav wird bei einem angelegtem Druck von 10-12 bar mit Argon gefüllt und wieder entladen. Diese Operation wird viermal durchgeführt. Nun werden 0,5 g (2,14 mmol) des gemäss Beispiel A.2 erhaltenen (*Z*)-(2*R*)-[3-(3-Methoxy-phenyl)-2-methyl-pent-3-enyl]-dimethyl-amins_und 5 ml frisch destilliertes Ethanol in einem 10 ml-Schlenkgefäss mit Magnetrührer plaziert und dieses sechsmal einer Sequenz aus Anlegen von einem hohem Vakuum und entspannen mit Argon ausgesetzt. Die Lösung wird mit Hilfe eines Eisbades auf 0°C abgekühlt und anschliessend vorsichtig 70 µl (1,07 mmol) Methansulfonsäure zugegeben. In einem weiterem, nach obigem Verfahren unter Argon gesetztem, 10 ml-Schlenkgefäss werden [Rh(nbd)₂] BF₄ (3,2 mg; 0,0086 mmol) und (S)-Solphos (Ligand A, 6,0 mg; 0,0090 mmol) gegeben und in 5 ml Ethanol gelöst. Beide Lösungen werden für 10 min bei RT gerührt und anschliessend mittels Kanülen und leichtem Argonstrom in den Autoklaven überführt. Der Autoklav wird mit Wasserstoffgas gefüllt (10 bar, viermal) und schliesslich Wasserstoffgas bei 10 bar aufgepresst. Die Temperatur wird bei 25°C gehalten und Rühren gestartet. Nach 67,5 h Reaktionszeit wird der Druck auf Normaldruck entspannt. Es wird eine klare Lösung erhalten. Das Produkt wird mittels Rotationsverdampfer bei etwa 40°C Badtemperatur zur Trockene eingeengt. Die Reinheit wird mittels GC zu 94,1% ermittelt und das Diastereomerenverhältnis von (2*R*,3*R*) zu (2*R*,3*S*) respektive trans:cis-Verhältnis wird zu 91,7 : 8,3, respektive 11,1 : 1 bestimmt.

### Beispiel B2:

Die Ergebnisse des Beispiels B1 und weiterer analog dem Verfahren gemäss Beispiel B1 durchgeführter Experimente sind in der nachfolgenden Tabelle 1 zusammengefasst.

**Tabelle 1:**

| Katalysator / Ligand | pH₂ [bar] | Zeit [h] | Umsatz [%] | cis [%] | trans [%] |
|---|---|---|---|---|---|
| [Rh(nbd)₂] BF₄ / (S)-A | 10 | 67,5 | 94,1 | 8,3 | 91,7 |
| [Rh(nbd)₂] BF₄ / (S)-A | 20 | 16,5 | 37,0 | 10,4 | 89,6 |
| [Rh(nbd)₂] BF₄ / (R)-A | 20 | 16,5 | 50,1 | 11,5 | 88,5 |
| [Rh(nbd)₂] BF₄ / (R)-A | 20 | 67,5 | 77,8 | 12,5 | 87,5 |
| [Rh(nbd)₂] BF₄ / (R)-(S)-B | 20 | 19,0 | 100 | 13,4 | 86,6 |
| [Rh(nbd)₂] BF₄ / (R)-(S)-B | 20 | 20,0 | 97,6 | 18,5 | 81,5 |
| [Rh(nbd)₂] BF₄ / (S)-(R)-B | 20 | 20,0 | 13,7 | 25,0 | 75,0 |
| [Rh(nbd)₂] BF₄ / C | 20 | 20,0 | 41,5 | 14,5 | 85,5 |
| [Rh(nbd)₂] BF₄ / C | 20 | 18,5 | 22,7 | 15,4 | 84,6 |
| [Rh(nbd)₂] BF₄ / D | 20 | 20,0 | 4,0 | 16,7 | 83,3 |
| [Rh(nbd)₂] BF₄ / E | 20 | 20,0 | 31,0 | 17,5 | 82,5 |
| [Rh(nbd)₂] BF₄ / F | 20 | 20,0 | 6,2 | 21,4 | 78,6 |
| [Rh(nbd)₂] BF₄ / (+)-G | 20 | 18,0 | 29,7 | 91,0 | 9,0 |
| [Rh(COD)₂] BF₄ / (S)-(S)-H | 20 | 16,5 | 96,6 | 96,3 | 3,7 |
| [Rh(nbd)₂] BF₄ / (S,S,S,S)-I | 20 | 19,0 | 100,0 | 75,2 | 24,8 |

Liganden: Die Strukturen der verwendeten Liganden sind nachfolgend abgebildet:

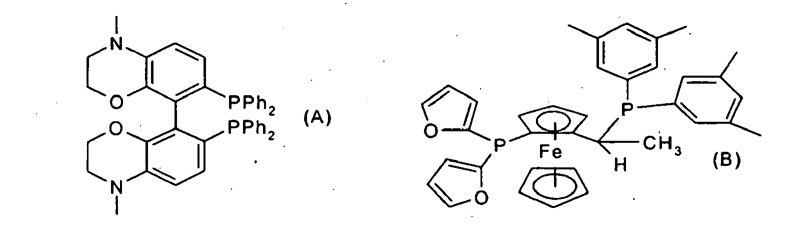

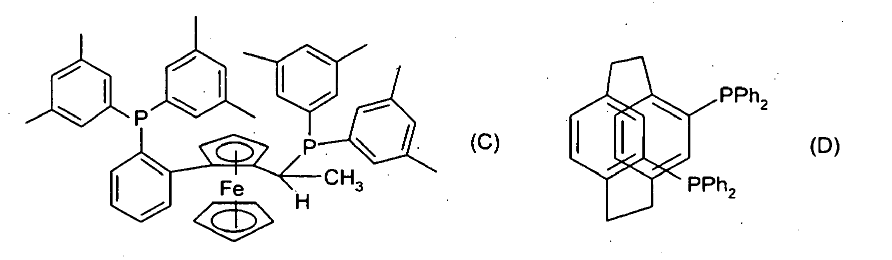

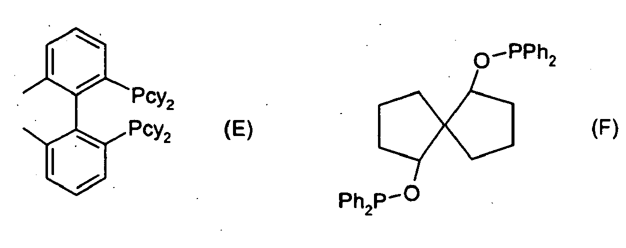

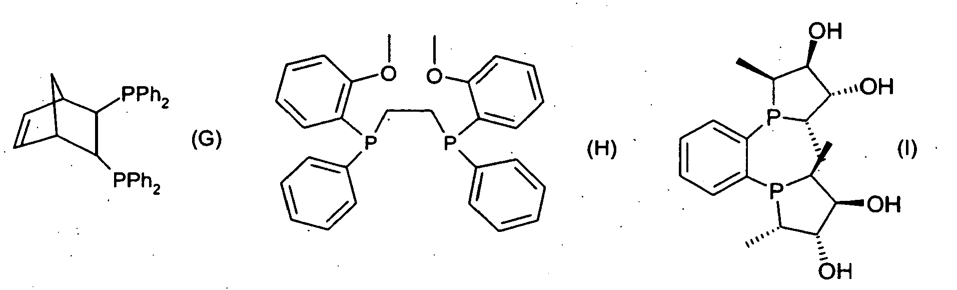

### Anwerkung:

Über die Hydrierung von homoallylischen Aminen ist nur wenig bekannt. Erstaunlicherweise gelingt es jedoch mit der homogenen Katalyse durch Variation des Katalysators selektiv beide Diastereomeren (2*R*, 3*R*)- [3-(3-Methoxy-phenyl)-2-methyl-pentyl]-dimethyl-amin und (2*R*, 3*S*)-[3-(3-Methoxy-phenyl)-2-methyl-pentyl]-dimethyl-amin in hoher Ausbeute und optischer Reinheit herzustellen.

Vorteilhaft für die erfolgreiche hochselektive Hydrierung ist in der Regel der Einsatz von gereinigtem Ausgangsprodukt. Dazu wird das aus der Eliminierung anfallende Gemisch von (*Z*)-(2*R*)-[3-(3-Methoxy-phenyl)-2-methyl-pent-3-enyl]-dimethyl-amin, (*E*)-(2*R*)-[3-(3-Methoxyphenyl)-2-methyl-pent-3-enyl]-dimethyl-amin und weiteren unbekannten Nebenprodukten einer Hydrochloridfällung unterzogen.

Im Fall der Hydrierung von (*Z*)-(2*R*)-[3-(3-Methoxy-phenyl)-2-methyl-pent-3-enyl]-dimethylamin mit einem homogenen Rhodiumkatalysator enthaltend den Liganden (*S*)-A wird eine Reinheit von 94,1% und ein Diastereomerenverhältnis der (2*R*,3*R*)- zur (2*R*,3*S*)- Verbindung von 11,1:1 erreicht. Erstaunlicherweise liefert auch die Hydrierung mit einem Rhodiumkatalysator, der das andere Enantiomere des Liganden A, nämlich (*R*)-A enthält, fast gleiche Diasteromerenverhältnisse wie bei dem Liganden (*S*)-A. Es kann daher angenommen werden, dass auch ein Katalysator mit racemischem Ligand gute Ergebnisse erbringt, was einen erheblichen wirtschaftlichen Vorteil darstellt.

Wird der Ligand H eingesetzt, wird das andere Diastereomere, die (2*R*,3*S*)-Verbindung mit einer Reinheit von etwa 97% und einem Diastereomerenverhältnis von 29:1 (97%) erhalten.

## Patentansprüche

1. Verfahren zur Herstellung wenigstens einer substituierten Dimethyl-(3-aryl-butyl)-amin-Verbindung der allgemeinen Formel III, 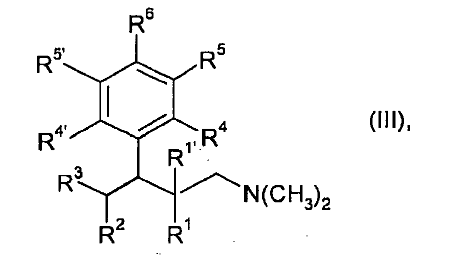 worin
R¹, R^{1'}, R², R³ unabhängig voneinander jeweils -H oder -C₁₋₅-Alkyl bedeuten,
R⁴, R^{4'}, R⁵, R^{5'}, R⁶, gleich oder verschieden, jeweils für -H, -OH, -C₁₋₄-Alkyl, -O-C₁₋₄ Alkyl, teil- oder perfluoriertes -C₁₋₄-Alkyl, teil- oder perfluoriertes -O-C₁₋₄-Alkyl, -O-(CH₂)ₙ-Phenyl mit n gleich 1, 2 oder 3, F, Cl oder OR⁸ stehen, oder zwei benachbarte Reste R⁴ und R⁵, R⁵ und R⁶, R⁶ und R^{5'} oder R^{5'} und R^{4'} für eine Gruppe -OCH=CHO, -CH=C(R⁹)-O-, -CH=C(R⁹)-S- oder -CH=CH-C(OR¹⁰)=CH- als Teil eines Ringes stehen, mit der Massgabe, dass die jeweils anderen Reste R⁶, R⁵ und R^{4'}, R⁴, R^{5"} und R^{6'}, R⁴, R⁵ und R^{4'} bzw. R⁴, R⁵ und R⁶ die vorstehend genannte Bedeutung haben,
R⁸ -CO-C₁₋₅-Alkyl, -PO(O-C₁₋₄-Alkyl)₂, -CO-C₆H₄-R¹¹, -CO(O-C₁₋₅-Alkyl), -CO-CHR¹²-NHR¹³, -CO-NH-C₆H₃-(R¹⁴)₂ oder eine unsubstituierte oder substituierte Pyridyl-, Thienyl-, Thiazoyl- oder Phenylgruppe bedeutet,
R⁹ -H oder -C₁₋₄-Alkyl bedeutet,
R¹⁰ -H oder -C₁₋₃-Alkyl bedeutet,
R¹¹ -OC(O)-C₁₋₃-Alkyl in ortho-Stellung oder -CH₂-N-(R¹⁵)₂ in meta- oder para-Stellung, wobei R¹⁵ jeweils -C₁₋₄-Alkyl oder beide Reste R¹⁵ zusammen mit dem verbrückenden Stickstoffatom einen 4-Morpholino-Rest bilden,
R¹² und R¹³, gleich oder verschieden, jeweils für -H, -C₁₋₆-Alkyl oder -C₃₋₈-Cycloalkyl stehen,
oder R¹² und R¹³ zusammen -(CH₂)₃₋₈- als Teil eines Rings bedeuten,
R¹⁴ -H, -OH, -C₁₋₇-Alkyl, teil-oder perfluoriertes -C₁₋₇-Alkyl, -OC₁₋₇-Alkyl, -Phenyl, -O-Aryl, -F oder -Cl mit der Massgabe, dass die Reste R¹⁴ gleich oder verschieden sind,
jeweils in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, gegebenenfalls in Form eines Salzes, eines Solvates oder in Form eines Salzes und Solvates,
**dadurch gekennzeichnet, dass** man eine substituierte Dimethyl-(3-aryl-but-3-enyl)-amin-Verbindung der allgemeinen Formel II, 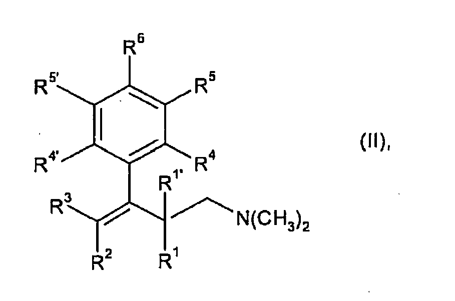 worin die Reste R¹, R^{1'}, R², R³, R⁴, R^{4'}, R⁵, R^{5'} und R⁶ jeweils die vorstehend genannte Bedeutung haben, jeweils in Form von Racematen, reinen Enantiomeren, Gemischen von Enantiomeren in einem beliebigen Mischungsverhältnis, Z- oder E-Isomeren oder Gemischen von Z- oder E-Isomeren in einem beliebigen Mischungsverhältnis, Salzen oder Solvaten, in Gegenwart von Wasserstoff und einem löslichen Rhodiumsalz oder Rhodiumkomplex als homogenem Katalysator zu einer Verbindung der allgemeinen Formel III umsetzt,
wobei die als Katalysator verwendeten Rhodiumsalze oder Rhodiumkomplexe chirale Liganden mit zwei Sekundärphosphingruppen (Diphosphinligand) aufweisen.

2. Verfahren gemäss Anspruch 1, **dadurch gekennzeichnet, dass** es sich bei der Verbindung der Formel II am solche der Formel IIA handelt, 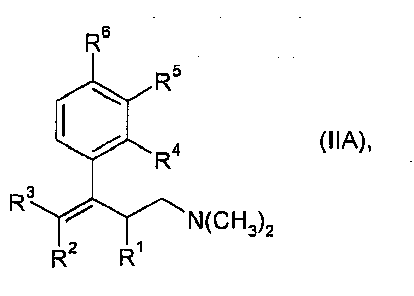 worin
R¹ -C₁₋₅-Alkyl ist,
R² -H oder -C₁₋₅-Alkyl bedeutet,
R³ -H oder -C₁₋₅-Alkyl bedeutet,
R⁴ -H, -OH, -C₁₋₄-Alkyl, -O-C₁₋₄-Alkyl, -O-Benzyl, -CF₃, -O-CF₃, -Cl, -F oder -OR⁸ ist,
R⁵ -H, -OH, -C₁₋₄-Alkyl, -O-C₁₋₄-Alkyl, -O-Benzyl, -CHF₂, -CF₃, -O-CF₃, -Cl, -F oder - OR⁸ ist,
R⁶ -H, -OH, -C₁₋₄-Alkyl, -O-C₁₋₄-Alkyl, -O-Benzyl, -CF₃, -O-CF₃, -Cl, -F oder -OR⁸ bedeutet,
mit der Massgabe, dass zwei der Reste R⁴, R⁵ oder R⁶ -H sind, oder R⁴ und R⁵ zusammen eine Gruppe -CH=C(R⁹)-O- oder -CH=C(R⁹)-S- als Teil eines Ringes bedeuten, mit der Massgabe, dass R⁶ -H ist, oder R⁵ und R⁶ zusammen eine Gruppe -CH=CH-C(OR¹⁰)=CH- als Teil eines Ringes bedeuten, mit der Massgabe, dass R⁴ - H ist, und
R⁸ -CO-C₁₋₅-Alkyl, -PO(O-C₁₋₄-Alkyl)₂, -CO-C₆H₄-R¹¹ -CO(O-C₁₋₅-Alkyl), -CO-CHR¹²-NHR¹³, -CO-NH-C₆H₃-(R¹⁴)₂ oder eine unsubstituierte oder substituierte Pyridyl-, Thienyl-, Thiazoyl- oder Phenylgruppe bedeutet,
R⁹ -H oder -C₁₋₄-Alkyl bedeutet, R¹⁰ -H oder C₁₋₃-Alkyl bedeutet,
R¹¹ -OC(O)- C₁₋₃-Alkyl in ortho-Stellung oder -CH₂-N-(R¹⁵)₂ in meta- oder para-Stellung,
wobei R¹⁵ -C₁₋₄-Alkyl ist oder beide Reste R¹⁵ zusammen mit dem verbrückenden Stickstoffatom einen 4-Morpholino-Rest bilden, bedeutet,
R¹² und R¹³, gleich oder verschieden, jeweils für -H, -C₁₋₆-Alkyl oder -C₃₋₈-Cycloalkyl stehen,
oder R¹² und R¹³ zusammen -(CH₂)₃₋₈ als Teil eines Rings bedeuten,
R¹⁴ -H, -OH, -C₁₋₇-Alkyl, -O-C₁₋₇-Alkyl, -Phenyl, -O-Aryl, -CF₃, -Cl oder -F bedeutet, mit der Massgabe, dass die beiden Reste R¹⁴ gleich oder verschieden sind.

3. Verfahren gemäss Anspruch 2, **dadurch gekennzeichnet, dass** in Formel IIA
R¹ -C₁₋₃-Alkyl,
R² -H oder C₁₋₃-Alkyl,
R³ -H oder C₁₋₃-Alkyl,
R⁴ -H, -OH, -Cl, -F oder -OR⁸,
R⁵ -H, -OH, -C₁₋₄-Alkyl, -O-C₁₋₄-Alkyl, -O-Benzyl, -CHF₂, -CF₃, -Cl, -F oder -OR⁸,
R⁶ -H, -OH, -O- C₁₋₄-Alkyl, -O-Benzyl, -CF₃, -Cl, -F oder -OR⁸ bedeutet,
mit der Massgabe, dass zwei der Reste R⁴, R⁵ oder R⁶ -H sind, oder R⁴ und R⁵ zusammen eine Gruppe -CH=C(R⁹)-O- oder -CH=C(R⁹)-S- als Teil eines Ringes bedeuten, mit der Massgabe, dass R⁶ -H ist, oder R⁵ und R⁶ zusammen eine Gruppe -CH=CH-C(OR¹⁰)=CH- als Teil eines Ringes bedeuten, mit der Massgabe, dass R⁴ - H ist, und
R⁸ bis R¹⁰ die in Anspruch 2 genannten Bedeutungen haben.

4. Verfahren gemäss Anspruch 3, **dadurch gekennzeichnet, dass** in Formel IIA
R¹ -CH₃ oder -C₃H₇ ist,
R² -H, -CH₃ oder -CH₂CH₃ ist,
R³ -H, -CH₃ oder -CH₂CH₃ ist,
R⁴ -H oder -OH ist,
^{R5} -H, -OH, -OCH₃, -CHF₂ oder -OR⁸ ist,
R⁶ -H, -OH oder -CF₃ ist,
mit der Massgabe, dass zwei Reste R⁴, R⁵ oder R⁶ -H sind, oder R⁴ und R⁵ zusammen eine Gruppe -CH=C(CH₃)-S- als Teil eines Ringes darstellen, mit der Massgabe, dass R⁶ -H ist, oder R⁵ und R⁶ zusammen -CH=CH-C(OH)=CH- als Teil eines Ringes bedeuten mit der Massgabe, dass R⁴ -H ist, R⁸ -CO-C₆H₄-R¹¹ und R¹¹ - OC(O)-C₁₋₃-Alkyl in ortho-Stellung bedeutet.

5. Verfahren gemäss Anspruch 4, **dadurch gekennzeichnet, dass** in Formel IIA R¹ und R³ jeweils -CH₃ und R⁵ -OCH₃ und die übrigen Reste ein Wasserstoffatom darstellen, entsprechend der Verbindung der Formel IIB: 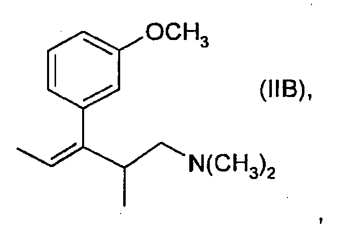 in Form von Mischungen seiner Stereoisomeren oder in Form eines spezifischen, isolierten Stereoisomers eingesetzt werden.

6. Verfahren gemäss Anspruch 5, **dadurch gekennzeichnet, dass** die Verbindung der Formel IIB (*Z*)-(2R)-3-[(3-Methoxy-phenyl)-2-methyl-pent-3-enyl]-dimethylamin ist.

7. Verfahren gemäss Anspruch 6, **dadurch gekennzeichnet, dass** die Verbindung der Formel III Diastereomere (2*R*,3*R*)- und (2*S*,3*R*)-[3-(3-Methoxy-phenyl)-2-methylpentyl]-dimethyl-amin sind, wobei das Diastereomerenverhältnis bei grösser oder gleich 70 : 30 zugunsten eines der beiden Diastereomere liegt.

8. Verfahren gemäss Anspruch 1, **dadurch gekennzeichnet, dass** der Ligand der Formel Sekundärphosphin-Gerüst-Sekundärphosphin entspricht, der Ligand mit dem Metallatom einen fünf- bis zehngliedrigen Ring bildet, und das Gerüst 2 bis 30 C-Atome und gegebenenfalls Heteroatome O, S, NH und/oder N-C₁-C₄-Alkyl enthält.

9. Verfahren gemäss Anspruch 8, **dadurch gekennzeichnet, dass** es sich bei dem Gerüst um bivalente Reste von Alkanen, Heteroalkanen, Alkenen, Zykloalkanen, Zykloalkenen, Heterozykloalkanen, Heterozykloalkenen, Bizykloalkanen, Bizykloheteroalkanen, Spirobiszykloalkanen, Spirobiszykloheteroalkanen, Arylenen, Heteroarylenen, Bisrarylenen, Bisheteroarylenen, Metallocenen, bevorzugt Ferrocen handelt, und der Rest unsubstituiert oder mit C₁-C₆-Alkyl, C₁-C₆-Alkoxy, C₁-C₆-Alkylthio, C₄-C₈-Cycloalkyl, Phenyl, Benzyl, Phenoxy, Benzyloxy, Phenylthio, Benzylthio, Halogen, OH, Tri(C₁-C₆-Alkyl)silyl, Sekundäramino, -CO₂H, -SO₃H₁ - CO₂R', -SO₃R', -O-C(O)-R', -NH-C(O)R', -O-SO₃-R' und/oder -NH-SO₃R', substituiert ist, wobei R' für C₁-C₆-Alkyl, C₄-C₈-Cycloalkyl, Phenyl oder Benzyl steht.

10. Verfahren gemäss Anspruch 1, **dadurch gekennzeichnet, dass** die sekundären Phosphingruppen gleiche oder verschiedene Kohlenwasserstoffreste als Substituenten enthalten und die beiden sekundären Phosphingruppen in den Diphosphinliganden gleich oder verschieden sind.

11. Verfahren gemäss Anspruch 1, **dadurch gekennzeichnet, dass** es sich bei den Diphosphinliganden um solche der Formeln A bis I handelt, 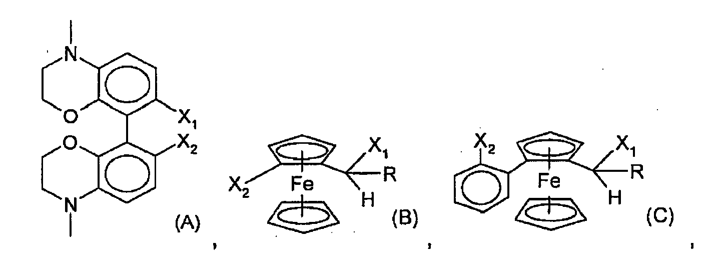 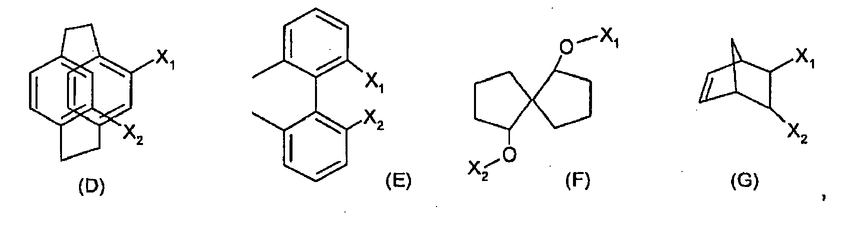 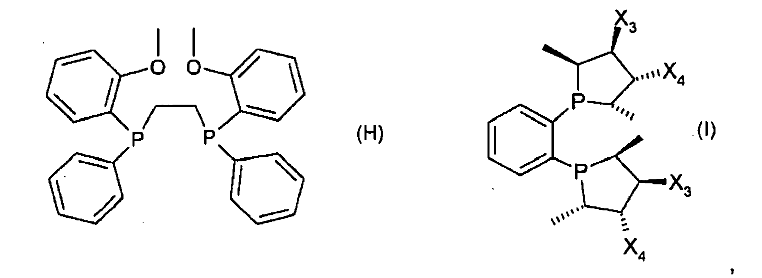 worin bedeuten:
X₁ und X₂ unabhängig voneinander ein Sekundärphosphin, worin die Phosphingruppe zwei gleiche oder verschiedene Reste, ausgewählt aus der Gruppe lineares oder verzweigtes C₁-C₁₂-Alkyl; unsubstituiertes oder mit C₁-C₆-Alkyl oder C₁-C₆-Alkoxy substituiertes C₅-C₁₂-Cycloalkyl oder C₅-C₁₂-Cycloalkyl-CH₂₋₁ Phenyl, Naphthyl, Furyl oder Benzyl; oder mit Halogen, C₁-C₆-Alkyl, Trifluormethyl, C₁-C₆-Alkoxy, Trifluormethoxy, (C₆H₅)₃Si, (C₁-C₁₂-Alkyl)₃Si, oder Sekundäramino substituiertes Phenyl oder Benzyl,
R C₁-C₄-Alkyl, bevorzugt Methyl, C₆-C₁₀-Aryl, oder C₇-C₁₂-Aralkyl, und
X₃ und X₄ unabhängig voneinander H, OH, C₁-C₄-Alkyl, C₁-C₄-Alkoxy, Benzyloxy, - CH(C₁-C₄-Alkyl)- oder -CH(Phenyl)-, vorzugsweise je -OH.

12. Verfahren gemäss Anspruch 11, **dadurch gekennzeichnet, dass** man eine Verbindung der Formel IIC.1 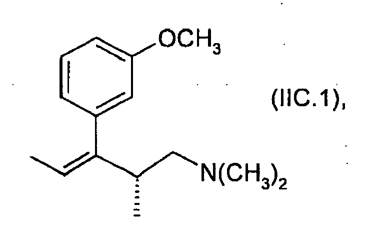 einsetzt, der Diphosphinligand ausgewählt ist aus einer Verbindung der Formeln A bis F gemäss Anspruch 11, und als Verbindung der Formel III überwiegend das Diastereomere (2*R*,3*R*)-[3-(3-Methoxy-phenyl)-2-methyl-pentyl]-dimethyl-amin erhalten wird, wobei das Diastereomerenverhältnis (2R,3R):(2S,3R) bei grösser oder gleich 75 : 25 liegt.

13. Verfahren gemäss Anspruch 11, **dadurch gekennzeichnet, dass** man eine Verbindung der Formel IIC.1 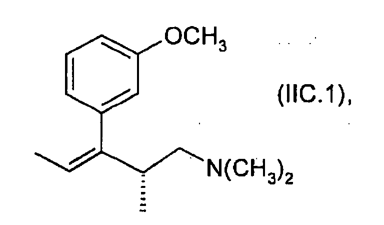 einsetzt, der Diphosphinligand ausgewählt ist aus einer Verbindung der Formeln G, H oder I gemäss Anspruch 11, und als Verbindung der Formel III überwiegend das Diastereomere (2*S*,3*R*)-[3-(3-Methoxy-phenyl)-2-methyl-pentyl]-dimethyl-amin erhalten wird, wobei das Diastereomerenverhältnis (2S,3R):(2R,3R) bei grösser oder gleich 75 : 25 liegt.

## Claims

1. Process for the preparation of at least one substituted dimethyl-(3-aryl-butyl)-amine compound of the general formula III 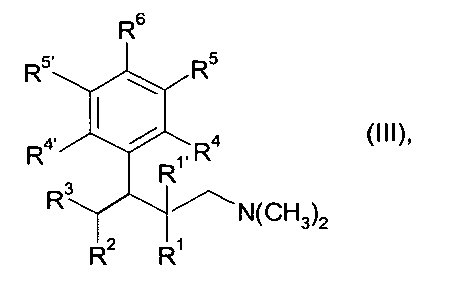 wherein
R¹, R^{1'}, R², R³ independently of one another each denote -H or -C₁₋₅-alkyl,
R⁴, R^{4'}, R⁵, R^{5'}, R⁶ are identical or different and each represent -H, -OH, -C₁₋₄-alkyl, -O-C₁₋₄-alkyl, partly fluorinated or perfluorinated -C₁₋₄-alkyl, partly fluorinated or perfluorinated -O-C₁₋₄-alkyl, -O-(CH₂)ₙ-phenyl where n is 1, 2 or 3, F, Cl or OR⁸, or two adjacent radicals R⁴ and R⁵, R⁵ and R⁶, R⁶ and R^{5'} or R^{5'} and R^{4'} represent a group -OCH=CHO-, -CH=C(R⁹)-O-, -CH=C(R⁹)-S- or -CH=CH-C(OR¹⁰)=CH- as part of a ring, with the proviso that the other particular radicals R⁶, R⁵ and R^{4'}, R⁴, R^{5'} and R^{6'}, R⁴, R⁵ and R^{4'} or R⁴, R⁵ and R⁶ have the abovementioned meaning,
R⁸ denotes -CO-C₁₋₅-alkyl, -PO(O-C₁₋₄-alkyl)₂, -CO-C₆H₄-R¹¹, -CO(O-C₁₋₅-alkyl), -CO-CHR¹²-NHR¹³, -CO-NH-C₆H₃-(R¹⁴)₂ or an unsubstituted or substituted pyridyl, thienyl, thiazolyl or phenyl group,
R⁹ denotes -H or -C₁₋₄-alkyl,
R¹⁰ denotes -H or -C₁₋₃-alkyl,
R¹¹ -OC(O)-C₁₋₃-alkyl in the ortho-position or -CH₂-N-(R¹⁵)₂ in the meta- or para-position, wherein R¹⁵ in each case -C₁₋₄-alkyl or the two radicals R¹⁵ together with the bridging nitrogen atom form a 4-morpholino radical,
R¹² and R¹³ are identical or different and each represent -H, -C₁₋₆-alkyl or -C₃₋₈-cycloalkyl,
or R¹² and R¹³ together denote -(CH₂)₃₋₈- as part of a ring,
R¹⁴ -H, -OH, -C₁₋₇-alkyl, partly fluorinated or perfluorinated -C₁₋₇-alkyl, -OC₁₋₇-alkyl, -phenyl, -O-aryl, -F or -Cl, with the proviso that the radicals R¹⁴ are identical or different,
in each case in the form of one of their pure stereoisomers, in particular enantiomers or diastereomers, their racemates or in the form of a mixture of stereoisomers, in particular the enantiomers or diastereomers, in any desired mixing ratio, optionally in the form of a salt, a solvate or in the form of a salt and solvate,
**characterized in that** a substituted dimethyl-(3-aryl-but-3-enyl)-amine compound of the general formula II 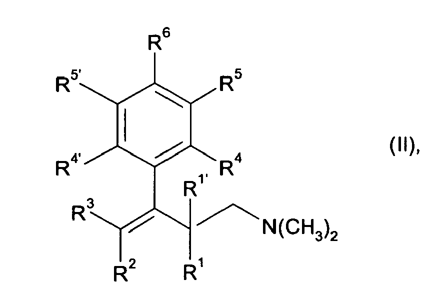 wherein the radicals R¹, R^{1'}, R², R³, R⁴, R⁴', R⁵, R^{5'} and R⁶ each have the abovementioned meaning, in each case in the form of racemates, pure enantiomers, mixtures of enantiomers in any desired mixing ratio, Z or E isomers or mixtures of Z or E isomers in any desired mixing ratio, salts or solvates, is reacted in the presence of hydrogen and a soluble rhodium salt or rhodium complex, as a homogeneous catalyst, to give a compound of the general formula III,
wherein the rhodium salts or rhodium complexes used as the catalyst contain chiral ligands with two secondary phosphine groups (diphosphine ligand).

2. Process according to claim 1, **characterized in that** the compound of the formula II is one of the formula IIA 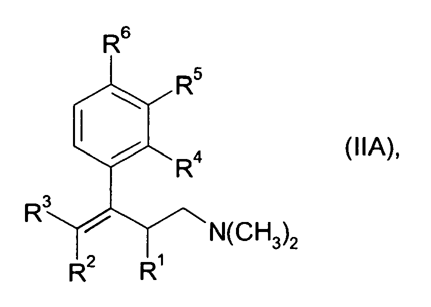 wherein
R¹ is -C₁₋₅-alkyl,
R² denotes -H or -C₁₋₅-alkyl,
R³ denotes -H or -C₁₋₅-alkyl,
R⁴ is -H, -OH, -C₁₋₄-alkyl, -O-C₁₋₄-alkyl, -O-benzyl, -CF₃, -O-CF₃, -Cl, -F or -OR⁸,
R⁵ is -H, -OH, -C₁₋₄-alkyl, -O-C₁₋₄-alkyl, -O-benzyl, -CHF₂, -CF₃, -O-CF₃, -Cl, -F or -OR⁸,
R⁶ denotes -H, -OH, -C₁₋₄-alkyl, -O-C₁₋₄-alkyl, -O-benzyl, -CF₃, -O-CF₃, -Cl, -F or -OR⁸,
with the proviso that two of the radicals R⁴, R⁵ or R⁶ are -H, or R⁴ and R⁵ together denote a group -CH=C(R⁹)-O- or -CH=C(R⁹)-S- as part of a ring, with the proviso that R⁶ is -H, or R⁵ and R⁶ together denote a group -CH=CH-C(OR¹⁰)=CH- as part of a ring, with the proviso that R⁴ is -H, and
R⁸ denotes -CO-C₁₋₅-alkyl, -PO(O-C₁₋₄-alkylh, -CO-C₆H₄-R¹¹, -CO(O-C₁₋₅-alkyl), -CO-CHR¹²-NHR¹³, -CO-NH-C₆H₃-(R¹⁴)₂ or an unsubstituted or substituted pyridyl, thienyl, thiazolyl or phenyl group,
R⁹ denotes -H or -C₁₋₄-alkyl, R¹⁰ denotes -H or C₁₋₃-alkyl,
R¹¹ denotes -OC(O)-C₁₋₃-alkyl in the ortho-position or -CH₂-N-(R¹⁵)₂ in the meta- or para-position,
wherein R¹⁵ is -C₁₋₄-alkyl or the two radicals R¹⁵ together with the bridging nitrogen atom form a 4-morpholino radical,
R¹² and R¹³ are identical or different and each represent -H, -C₁₋₆-alkyl or -C₃₋₈-cycloalkyl,
or R¹² and R¹³ together denote -(-CH₂)₃₋₈ as part of a ring,
R¹⁴ denotes -H, -OH, -C₁₋₇-alkyl, -O-C₁₋₇-alkyl, -phenyl, -O-aryl, -CF₃, -Cl or -F, with the proviso that the two radicals R¹⁴ are identical or different.

3. Process according to claim 2, **characterized in that** in formula IIA
R¹ denotes -C₁₋₃-alkyl,
R² denotes -H or C₁₋₃-alkyl,
R³ denotes -H or C₁₋₃-alkyl,
R⁴ denotes -H, -OH, -Cl, -F or -OR⁸,
R⁵ denotes -H, -OH, -C₁₋₄-alkyl, -O-C₁₋₄-alkyl, -O-benzyl, -CHF₂, -CF₃, -Cl, -F or -OR⁸,
R⁶ denotes -H, -OH, -O- C₁₋₄-alkyl, -O-benzyl, -CF₃, -Cl, -F or -OR⁸,
with the proviso that two of the radicals R⁴, R⁵ or R⁶ are -H, or R⁴ and R⁵ together denote a group -CH=C(R⁹)-O- or -CH=C(R⁹)-S- as part of a ring, with the proviso that R⁶ is -H, or R⁵ and R⁶ together denote a group -CH=CH-C(OR¹⁰)=CH- as part of a ring, with the proviso that R⁴ is -H, and
R⁸ to R¹⁰ have the meanings given in claim 2.

4. Process according to claim 3, **characterized in that** in formula IIA
R' is -CH₃ or -C₃H₇,
R² is -H, -CH₃ or -CH₂CH₃,
R³ is -H, -CH₃ or -CH₂CH₃,
R⁴ is -H or -OH,
R⁵ is -H, -OH, -OCH₃, -CHF₂ or -OR⁸,
R⁶ is -H, -OH or -CF₃,
with the proviso that two radicals R⁴, R⁵ or R⁶ are -H, or R⁴ and R⁵ together represent a group -CH=C(CH₃)-S- as part of a ring, with the proviso that R⁶ is -H, or R⁵ and R⁶ together denote -CH=CH-C(OH)=CH- as part of a ring, with the proviso that R⁴ is -H, R⁸ denotes -CO-C₆H₄-R¹¹ and R¹¹ denotes -OC(O)-C₁₋₃-alkyl in the ortho-position.

5. Process according to claim 4, **characterized in that** in formula IIA, R¹ and R³ each represent -CH₃ and R⁵ represents -OCH₃ and the remaining radicals represent a hydrogen atom, corresponding to the compound of the formula IIB: 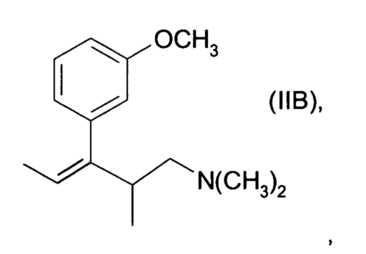 are employed in the form of mixtures of its stereoisomers or in the form of a specific, isolated stereoisomer.

6. Process according to claim 5, **characterized in that** the compound of the formula IIB is (Z)-(2*R*)-3-[(3-methoxy-phenyl)-2-methyl-pent-3-enyl]-dimethylamine.

7. Process according to claim 6, **characterized in that** the compound of the formula III is the diastereomers (2*R*,3*R*)- and (2*S*,3*R*)-[3-(3-methoxy-phenyl)-2-methyl-pentyl]-dimethyl-amine, wherein the diastereomeric ratio is greater than or equal to 70 : 30 in favour of one of the two diastereomers.

8. Process according to claim 1, **characterized in that** the ligand corresponds to the formula secondary phosphine-skeleton-secondary phosphine, the ligand forms a five- to tenmembered ring with the metal atom, and the skeleton contains 2 to 30 C atoms and optionally hetero atoms O, S, NH and/or N-C₁-C₄-alkyl.

9. Process according to claim 8, **characterized in that** the skeleton is bivalent radicals of alkanes, heteroalkanes, alkenes, cycloalkanes, cycloalkenes, heterocycloalkanes, heterocycloalkenes, bicycloalkanes, bicycloheteroalkanes, spirobiscycloalkanes, spirobiscycloheteroalkanes, arylenes, heteroarylenes, bisarylenes, bisheteroarylenes, metallocenes, preferably ferrocene, and the radical is unsubstituted or substituted by C₁-C₆-alkyl, C₁-C₆-alkoxy, C₁-C₆-alkylthio, C₄-C₈-cycloalkyl, phenyl, benzyl, phenoxy, benzyloxy, phenylthio, benzylthio, halogen, OH, tri(C₁-C₆-alkyl)silyl, secondary amino, -CO₂H, -SO₃H, -CO₂R', -SO₃R', -O-C(O)-R', -NH-C(O)R', -O-SO₃-R', and -NH-SO₃R', wherein R' represents C₁-C₆-alkyl, C₄-C₈-cycloalkyl, phenyl or benzyl.

10. Process according to claim 1, **characterized in that** the secondary phosphine groups contain identical or different hydrocarbon radicals as substituents, and the two secondary phosphine groups in the diphosphine ligands are identical or different.

11. Process according to claim 1, **characterized in that** the diphosphine ligands are those of the formulae A to I 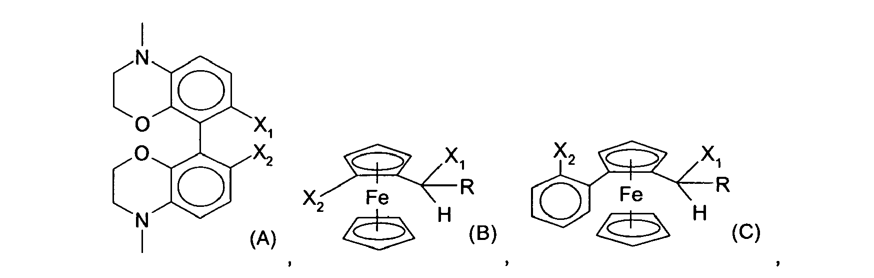 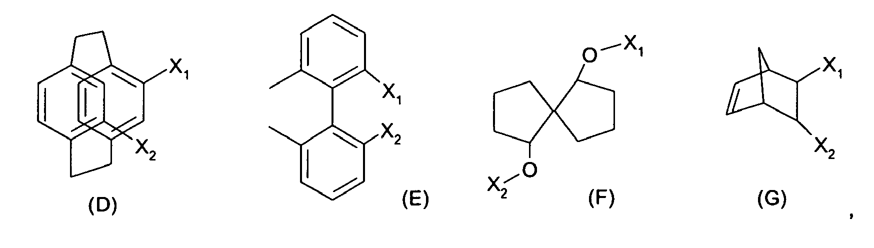 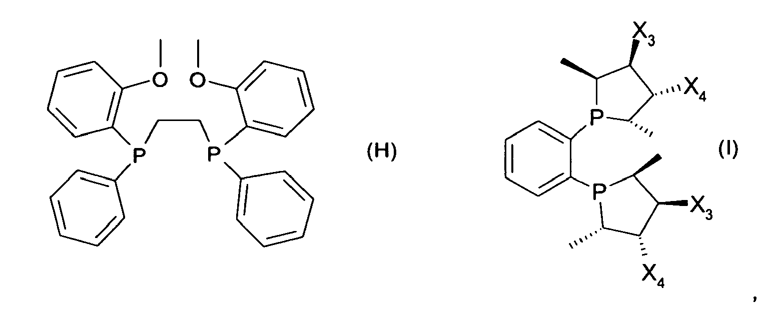 wherein:
X₁ and X₂ independently of one another denote a secondary phosphine, wherein the phosphine group two identical or different radicals chosen from the group of linear or branched C₁-C₁₂-alkyl; C₅-C₁₂-cycloalkyl or C₆-C₁₂-cycloalkyl-CH₂- which is unsubstituted or substituted by C₁-C₆-alkyl or C₁-C₆-alkoxy; phenyl, naphthyl, furyl or benzyl; or phenyl or benzyl which is substituted by halogen, C₁-C₆-alkyl, trifluoromethyl, C₁-C₆-alkoxy, trifluoromethoxy, (C₆H₅)₃Si, (C₁-C₁₂-alkyl)₃Si or secondary amino,
R denotes C₁-C₄-alkyl, preferably methyl, C₆-C₁₀-aryl, or C₇-C₁₂-aralkyl, and
X₃ and X₄ independently of one another denote H, OH, C₁-C₄-alkyl, C₁-C₄-alkoxy, benzyloxy, -CH(C₁-C₄-alkyl)- or -CH(phenyl)-, preferably in each case -OH.

12. Process according to claim 11, **characterized in that** a compound of the formula IIC.1 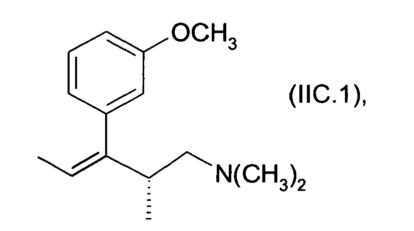 is employed, the diphosphine ligand is chosen from a compound of the formulae A to F according to claim 11, and the diastereomer (2*R*,3*R*)-[3-(3-methoxy-phenyl)-2-methyl-pentyl]-dimethyl-amine is predominantly obtained as the compound of the formula III, wherein the diastereomeric ratio (2R,3R):(2S,3R) is greater than or equal to 75 : 25.

13. Process according to claim 11, **characterized in that** a compound of the formula IIC.1 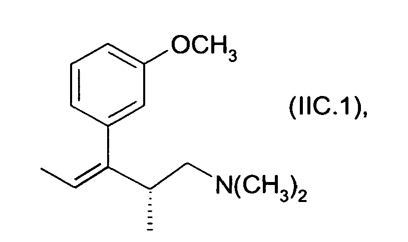 is employed, the diphosphine ligand is chosen from a compound of the formulae G, H or I according to claim 11, and the diastereomer (2*S*,3*R*)-[3-(3-methoxy-phenyl)-2-methyl-pentyl]-dimethyl-amine is predominantly obtained as the compound of the formula III, wherein the diastereomeric ratio (2S,3R):(2R,3R) is greater than or equal to 75 : 25.

## Revendications

1. Procédé pour la préparation d'au moins un composé substitué de diméthyl-(3-arylbutyl)-amine de formule générale III, 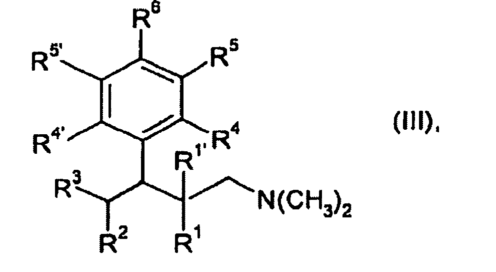 où
R¹, R^{1'}, R², R³ signifient, indépendamment l'un de l'autre, à chaque fois -H ou -C₁₋₅-alkyle ;
R⁴ R^{4'}, R⁵ R^{5'}, R⁶, sont identiques ou différents et représentent à chaque fois -H, -OH, -C₁₋₄-alkyle, -O-C₁₋₄-alkyle, -C₁₋₄-alkyle partiellement fluoré ou perfluoré, -O-C₁₋₄-alkyle partiellement fluoré ou perfluoré, -O-(CH₂)ₙ-phényle avec n valant 1, 2 ou 3, F, C1 ou OR⁸, ou deux radicaux adjacents R⁴ et R⁵, R⁵ et R⁶, R⁶ et R^{5'} ou R^{5'} et R^{4'} représentent un groupe -OCH=CHO, -CH=C (R⁹) -O-, -CH=C (R⁹) -S- ou -CH=CH-C (OR¹⁰) =CH-comme partie d'un cycle, à condition qu'à chaque fois les autres radicaux R⁶, R⁵ et R^{4'}, R⁴, R^{5"} et R^{6'}, R⁹, R⁵ et R^{4'} ou, selon le cas, R⁴, R⁵ et R⁶ présentent la signification susmentionnée,
R⁸ signifie -CO-C₁₋₅-alkyle, -PO (O-C₁₋₉-alkyle)₂, -CO-C₆H₉-R¹¹, -CO(O-C₁₋₅-alkyle), -CO-CHR¹²-NHR¹³, -CO-NH-C₆H₃- (R¹⁴)₂ or un groupe pyridyle, thiényle, thiazoyle ou phényle non substitué ou substitué,
R⁹ signifie -H ou -C₁₋₄-alkyle,
R¹⁰ signifie -H ou -C₁₋₃-alkyle,
R¹¹ signifie -OC (O) -C₁₋₃-alkyle en position ortho ou -CH₂-N- (R¹⁵) ₂ en position méta ou para, où R¹⁵ représente à chaque fois -C₁₋₄-alkyle ou les deux radicaux R¹⁵ forment ensemble avec l'atome d'azote formant un pont entre eux un radical 4-morpholino,
R¹² et R¹³, identiques ou différents, représentent à chaque fois -H, -C₁₋₆-alkyle ou -C₃₋₈-cycloalkyle, ou
R¹² et R¹³ ensemble signifient -(CH₂)₃₋₈- comme partie d'un cycle,
R¹⁴ signifie -H, -OH, -C₁₋₇-alkyle, -C₁₋₇-alkyle partiellement fluoré ou fluoré, -OC₁₋₇-alkyle, -phényle, -O-aryle, -F ou -C1 à condition que les radicaux R¹⁴ soient identiques ou différents,
à chaque fois sous forme d'un de ses stéréo-isomères purs, en particulier ses énantiomères ou diastéréo-isomères, ses mélanges racémiques ou sous forme d'un mélange de stéréo-isomères, en particulier des énantiomères ou des diastéréo-isomères, dans un rapport de mélange quelconque, le cas échéant sous forme d'un sel, d'un solvate ou sous forme d'un sel et d'un solvate, **caractérisé en ce qu'**on transforme un composé substitué de diméthyl-(3-arylbut-3-ényl)-amine de formule générale II, 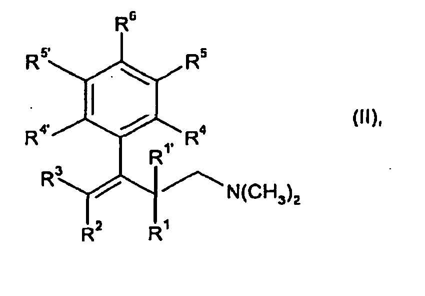 où les radicaux R^{1'}, R^{1'}, R², R³, R⁴, R^{4'}, R⁵, R^{5'} et R⁶ présentent à chaque fois la signification susmentionnée, à chaque fois sous forme de racémates, d'énantiomères purs, de mélanges d'énantiomères dans un rapport de mélange quelconque, d'isomères Z ou E ou de mélanges d'isomères Z ou E dans un rapport de mélange quelconque, de sels ou de solvates, en présence d'hydrogène et d'un sel ou d'un complexe de rhodium soluble comme catalyseur homogène en un composé de formule générale III,
où les sels ou complexes de rhodium utilisés comme catalyseur présentent des ligands chiraux avec deux groupes phosphine secondaire (ligand diphosphine).

2. Procédé selon la revendication 1, **caractérisé en ce qu'**il s'agit, pour le composé de formule II, de ceux de formule IIA, 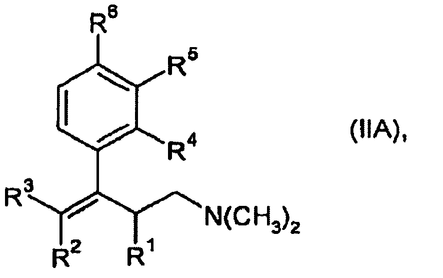 où
R¹ représente -C₁₋₅-alkyle,
R² signifie -H ou -C₁₋₅-alkyle,
R³ signifie -H ou -C₁₋₅-alkyle,
R⁴ représente -H, -OH, -C₁₋₄-alkyle, -O-C₁₋₄-alkyle, -O-benzyle, -CF₃, -O-CF₃, -Cl, -F ou -OR⁸,
R⁵ représente -H, -OH, -C₁₋₄-alkyle, -O-C₁₋₄-alkyle, -O-benzyle, -CHF₂, -CF₃, -O-CF₃, -Cl, -F ou -OR⁸,
R⁶ représente -H, -OH, -C₁₋₄-alkyle, -O-C₁₋₄-alkyle, -O-benzyle, -CF₃, -O-CF₃, -Cl, -F ou -OR⁸
à condition que deux des radicaux R⁴, R⁵ ou R⁶ représentent -H, ou R⁴ et R⁵ signifient, ensemble, un groupe -CH=C (R⁹) -O- ou -CH=C (R⁹) -S- comme partie d'un cycle, à condition que R⁶ représente -H, ou R⁵ et R⁶ signifient ensemble un groupe -CH=CH-C(OR¹⁰)=CH- comme partie d'un cycle, à condition que R⁴ représente -H, et
R⁸ signifie -CO-C₁₋₅-alkyle, -PO (O-C₁₋₄-alkyle) ₂, -CO-C₆H₄-R¹¹, -CO (O-C₁₋₅-alkyle) , -CO-CHR¹²-N_{HR}¹³, -CO-NH-C₆H₃- (R¹⁴) ₂ ou un groupe pyridyle, thiényle, thiazoyle ou phényle non substitué ou substitué,
R⁹ signifie -H ou -C₁₋₄-alkyle,
R¹⁰ signifie -H ou -C₁₋₃-alkyle,
R¹¹ signifie -OC (O) -C₁₋₃-alkyle en position ortho ou -CH₂-N- (R¹⁵)₂ en position méta ou para,
où
R¹⁵ représente -C₁₋₄-alkyle ou les deux radicaux R¹⁵ forment ensemble avec l'atome d'azote qui forme un pont entre eux un radical 4-morpholino,
R¹² et R¹³, identiques ou différents, représentent à chaque fois -H, -C₁₋₆-alkyle ou -C₃₋₈-cycloalkyle, ou
R¹² et R¹³ ensemble signifient - (CH₂) ₃₋₈ comme partie d'un cycle,
R¹⁴ signifie -H, -OH, -C₁₋₇-alkyle, -O-C₁₋₇-alkyle, -phényle, -O-aryle, -CF₃, -Cl ou -F, à condition que les deux radicaux R¹⁴ soient identiques ou différents.

3. Procédé selon la revendication 2, **caractérisé en ce que** dans la formule IIA
R¹ signifie -C₁₋₃-alkyle,
R² signifie -H ou C₁₋₃-alkyle,
R³ signifie -H ou C₁₋₃-alkyle,
R⁴ signifie -H, -OH, -Cl, -F ou -OR⁸,
R⁵ signifie -H, -OH, -C₁₋₄-alkyle, -O-C₁₋₄-alkyle, -O-benzyle, -CHF₂, -CF₃, -Cl, -F ou -OR⁸,
R⁶ signifie -H, -OH, -O-C₁₋₄-alkyle, -O-benzyle, -CF₃, -Cl, -F ou -OR⁸,
à condition que deux des radicaux R⁹, R⁵ ou R⁶ représentent -H, ou R⁴ et R⁵ signifient, ensemble, un groupe -CH=C(R⁹)-O- ou -CH=C (R⁹) -S- comme partie d'un cycle, à condition que R⁶ représente -H, ou R⁵ et R⁶ signifient ensemble un groupe -CH=CH-C(OR¹⁰)=CH- comme partie d'un cycle, à condition que R⁴ représente -H, et
R⁸ à R¹⁰ ont la signification mentionnée dans la revendication 2.

4. Procédé selon la revendication 3, **caractérisé en ce que** dans la formule IIA
R¹ représente -CH₃ ou -C₃H₇,
R² représente -H, -CH₃ ou -CH₂CH₃,
R³ représente -H, -CH₃ ou -CH₂CH₃,
R⁴ représente -H ou -OH,
R⁵ représente -H, -OH, -OCH₃, -CHF₂ ou -OR⁸,
R⁶ représente -H, -OH ou -CF₃,
à condition que deux des radicaux R⁴ R⁵ ou R⁶ représentent -H, ou R⁴ et R⁵ signifient, ensemble, un groupe -CH=C(CH₃)-S- comme partie d'un cycle, à condition que R⁶ représente -H, ou R⁵ et R⁶ signifient ensemble un groupe -CH=CH-C(OH)=CH- comme partie d'un cycle, à condition que R⁴ représente -H, R⁸ représente -CO-C₆H₄-R¹¹ et R¹¹ représente -OC(O)-C₁₋₃-alkyle en position ortho.

5. Procédé selon la revendication 4, **caractérisé en ce que** dans la formule IIA R¹ et R³ représentent à chaque fois -CH₃ et R⁵ représente -OCH₃ et les autres radicaux représentent un atome d'hydrogène, selon le composé de formule IIB : 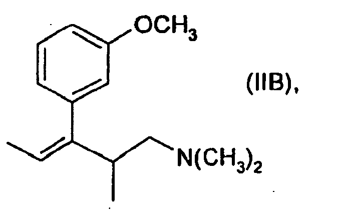 sous forme de mélanges de ses stéréo-isomères ou sous forme d'un stéréo-isomère spécifique, isolé.

6. Procédé selon la revendication 5, **caractérisé en ce que** le composé de formule IIB est la (Z)-(2R)-3-[(3-méthoxyphényl)-2-méthylpent-3-ényl]-diméthylamine.

7. Procédé selon la revendication 6, **caractérisé en ce que** le composé de formule III représente les diastéréo-isomères (2R,3R)-[3-(3-méthoxyphényl)-2-méthylpentyl]-diméthylamine et (2S,3R)-[3-(3-méthoxyphényl)-2-méthylpentyl]-diméthylamine, où le rapport des diastéréo-isomères est supérieur ou égal à 70:30 en faveur d'un des deux diastéréo-isomères.

8. Procédé selon la revendication 1, **caractérisé en ce que** le ligand correspond à la formule phosphine secondaire-structure-phosphine secondaire, le ligand forme un cycle de cinq à dix chaînons avec l'atome de métal, et la structure contient 2 à 30 atomes de carbone et le cas échéant des hétéroatomes O, S, NH et/ou N-C₁-C₄-alkyle.

9. Procédé selon la revendication 8, **caractérisé en ce qu'**il s'agit, pour la structure, de radicaux divalents d'alcanes, d'hétéroalcanes, d'alcènes, de cycloalcanes, de cycloalcènes, d'hétérocycloalcanes, d'hétérocycloalcènes, de bicycloalcanes, de bicyclohétéroalcanes, de spirobiscycloalcanes, de spirobiscyclohétéroalcanes, d'arylènes, d'hétéroarylènes, de bisarylènes, de bishétéroarylènes, de métallocènes, de préférence de ferrocène, et le radical est non substitué ou substitué par C₁-C₆-alkyle, C₁-C₆-alcoxy, C₁-C₆-alkylthio, C₄-C₈-cycloalkyle, phényle, benzyle, phénoxy, benzyloxy, phénylthio, benzylthio, halogène, OH, tri(C₁-C₆-alkyl)silyle, amino secondaire, -CO₂H -SO₃H, -CO₂R', -SO₃R', -O-C(O)-R', -NH-C(O)R', -O-SO₃-R' et/ou -NH-SO₃R', où R' représente C₁-C₆-alkyle, C₄-C₈-cycloalkyle, phényle ou benzyle.

10. Procédé selon la revendication 1, **caractérisé en ce que** les groupes phosphine secondaire contiennent des radicaux hydrocarbonés identiques ou différents comme substituants et les deux groupes phosphine secondaire dans les ligands de type diphosphine sont identiques ou différents.

11. Procédé selon la revendication 1, **caractérisé en ce qu'**il s'agit, pour les ligands de type diphosphine, de ceux des formules A à I : 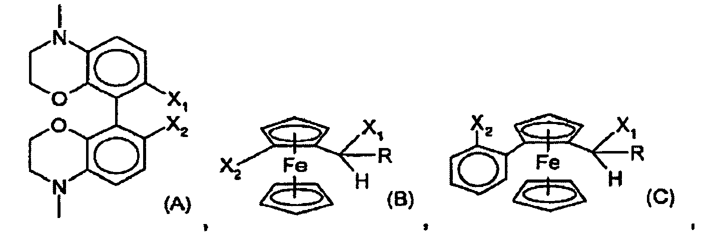 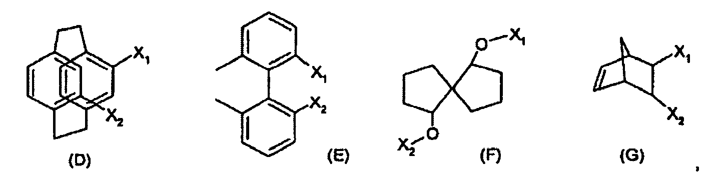 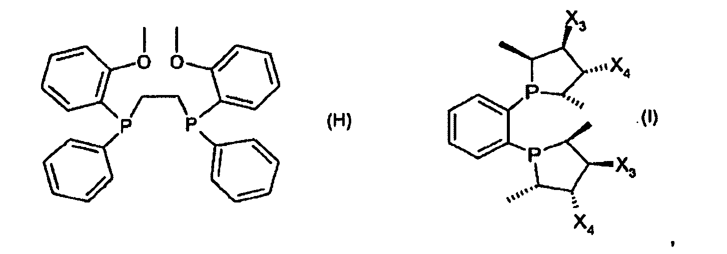 où
X₁ et X₂ signifient, indépendamment l'un de l'autre, une phosphine secondaire, où le groupe phosphine représente deux radicaux identiques ou différents, choisis dans le groupe formé par C₁-C₁₂-alkyle linéaire ou ramifié ; C₅-C₁₂-cycloalkyle ou C₅-C₁₂-cycloalkyl-CH₂- non substitué ou substitué par C₁-C₆-alkyle ou C₁-C₆-alcoxy ; phényle, naphtyle, furyle ou benzyle ; ou phényle ou benzyle substitué par halogène, C₁-C₆-alkyle, trifluorométhyle, C₁-C₆-alcoxy, trifluorométhoxy, (C₆H₅)₃Si, (C₁-C₁₂-alkyl)₃Si, ou amino secondaire,
R signifie C₁-C₄-alkyle, de préférence méthyle, C₆-C₁₀-aryle, ou C₇-C₁₂-aralkyle, et
X₃ et X₄ signifient, indépendamment l'un de l'autre, H, OH, C₁-C₄-alkyle, C₁-C₄-alcoxy, benzyloxy, -CH (C₁-C₄-alkyl)- ou -CH(phényl)-, de préférence chacun -OH.

12. Procédé selon la revendication 11, **caractérisé en ce qu'**on utilise un composé de formule IIC.1 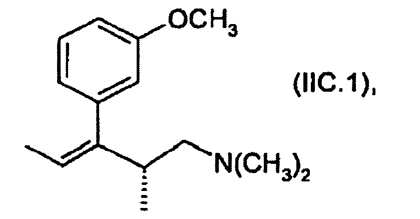 le ligand diphosphine est choisi parmi les composés des formules A à F selon la revendication 11, et on obtient comme composé de formule III principalement le diastéréo-isomère (2R,3R)-[3-(3-méthoxyphényl)-2-méthylpentyl]-diméthylamine, où le rapport des diastéréo-isomères (2R,3R):(2S,3R) est égal ou supérieur à 75:25.

13. Procédé selon la revendication 11, **caractérisé en ce qu'**on utilise un composé de formule IIC.1 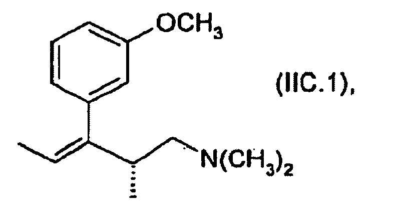 le ligand diphosphine est choisi parmi les composés des formules G, H ou I selon la revendication 11, et on obtient comme composé de formule III principalement le diastéréo-isomère (2S,3R)-[3-(3-méthoxyphényl)-2-méthylpentyl]-diméthylamine, où le rapport des
